(19) Europäisches Patentamt
European Patent Office
Office européen des brevets

(11) **EP 3 656 738 A1**

(12) **EUROPEAN PATENT APPLICATION**
published in accordance with Art. 153(4) EPC

(43) Date of publication:
**27.05.2020 Bulletin 2020/22**

(51) Int Cl.:
***C01B 33/18*** (2006.01)

(21) Application number: **17921127.1**

(86) International application number:
**PCT/JP2017/028922**

(22) Date of filing: **09.08.2017**

(87) International publication number:
**WO 2019/030853 (14.02.2019 Gazette 2019/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Wacker Chemie AG
81737 München (DE)**

(72) Inventors:
• **IGARASHI Kenji
Chikusei-shi
Ibaraki 300-4522 (JP)**
• **SENGOKU Airi
Chikusei-shi
Ibaraki 300-4522 (JP)**
• **ASAKAWA Yukihiko
Chikusei-shi
Ibaraki 300-4522 (JP)**

(74) Representative: **Mieskes, Klaus Theoderich et al
Wacker Chemie AG
Intellectual Property
Hanns-Seidel-Platz 4
81737 München (DE)**

(54) **AQUEOUS DISPERSION, METHOD FOR PRODUCING AQUEOUS DISPERSION, OIL-IN-WATER EMULSION, METHOD FOR PRODUCING OIL-IN-WATER EMULSION, AND DESIGN METHOD**

(57) Provided are an aqueous dispersion which exhibits properties required of an aqueous dispersion and achieves the stability and homogeneity of an aqueous dispersion, and a method for producing the aqueous dispersion. The aqueous dispersion of inorganic particles includes inorganic particle groups dispersed in an aggregation state in water. In this dispersion, when the total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is within a value range of a prescribed lower limit value or more depending on the aggregating properties of inorganic particles, the inorganic particle groups contain self-micelle-like aggregates and hydrophobic-rich aggregates.

Fig. 1

Hydrophilic-Rich Aggregate
(Soluble, Low Viscosity, Small Thixotropic Property)

Self-Micelle-Like Aggregate

Hydrophobic-Rich Aggregate
(High Viscosity, Large Thixotropic Property)

Homogeneity

Stability

EP 3 656 738 A1

**Description**

TECHNICAL FIELD

The present invention relates to an aqueous dispersion and a method for producing the aqueous dispersion, as well as an oil-in-water type emulsion, a method for producing and designing the oil-in-water type emulsion. More particularly, the present invention relates to an aqueous dispersion which exhibits properties required of an aqueous dispersion and achieves the stability and homogeneity of an aqueous dispersion, and a method for producing the aqueous dispersion, as well as an oil-in-water type emulsion which reliably achieves stability, a method for producing the oil-in-water type emulsion, and a method for designing the oil-in-water type emulsion which contributes to designing a practical emulsion product.

BACKGROUND ART

A known aqueous dispersion in which inorganic solid particles such as silica and titanium oxide are dispersed in water for achieving an emulsified state, a suspended state, and the like is widely used as a rheology control agent and a compounding agent to be formulated to cosmetics. In such an aqueous dispersion containing inorganic particles, the inorganic particles do not dissolve in water. Therefore, stability and homogeneity are important. The stability indicates that the inorganic particles are in a dispersion state and do not settle down for an extended period of time, and the homogeneity indicates that local unevenness does not exist in the aqueous dispersion which is in a dispersion state.
Particularly, when fumed silica is used as inorganic particles, and hydrophobic silica is used, increased viscosity and thixotropy are imparted to an aqueous dispersion, but an aqueous dispersion itself excessively increased in viscosity, and instability such as precipitation of hydrophobic silica particles is caused. On the other hand, when hydrophilic silica is used, hydrophilic silica dissolves in water to reliably achieve stability, but is not suitable for use applications which require a high viscosity as an aqueous dispersion.
As described above, required properties vary depending on use applications of an aqueous dispersion, and it has been technically difficult to simultaneously achieve both the required properties and the stability and the homogeneity of an aqueous dispersion.
In this regard, Patent Literature 1 discloses an aqueous dispersion containing silica as inorganic particles, in which attention has been paid on a contact angle of silica in a bulk state and such a contact angle is optimized to improve stability in which the precipitation of silica due to the passage of time is suppressed in the aqueous dispersion of silica.
However, it is not disclosed that the aqueous dispersion of Patent Literature 1 reliably achieves not only the stability in a dispersion state but also the homogeneity in a dispersion state. Needless to say, there is no suggestions as to whether properties required for the aqueous dispersion, and the stability and the homogeneity of the aqueous dispersion can be simultaneously achieved.
On the other hand, in an emulsion which is in an emulsified state between a solid phase or a liquid phase and a liquid phase as an aspect of a dispersion, a technology of using a surfactant to form an emulsified state has been known. It is also important for an emulsion, similarly to for an aqueous dispersion, to have stability in an emulsified state, in that a solid phase or a liquid phase and a liquid phase are not separated with the lapse of time.
In Patent Literature 2, in particular, a product obtained by emulsifying various oil ingredients with titanium oxide particles as a Pickering emulsion which includes inorganic particles as an alternative for a surfactant is used in cosmetics and the like use applications.
Furthermore, Patent Literature 3 discloses a known Pickering emulsion obtained by emulsifying a silicone oil and other silicone substances with silica particles.
Such a Pickering emulsion, as an oil-in-water type emulsion, includes composite particles which contain an oil droplet as a core and inorganic particles present on the surface of the oil droplet to serve as an interface between an oil phase and an aqueous phase. Such composite particles exist as a composite particle group in an emulsified state in an aqueous phase. This emulsion is formed without using an organic surfactant. Therefore, harmful effects by environmentally hazardous organic surfactants can be eliminated. Also, silica as a functional agent can be provided not as particles but as an aqueous system. In this regard, such a Pickering emulsion, as an oil-in-water type emulsion, is expected to be developed for various use applications such as medicines, food products, and defoamers.
It is pointed out in Pickering emulsions in Non-Patent Literature 1, Non-Patent Literature 2, and Non-Patent Literature 3 that a hydrophobicity-hydrophilicity balance of inorganic particles, as an alternative for a surfactant, present at an interface is important for reliably achieving the stability of the Pickering emulsion.
However, a known Pickering emulsion has the following technical problem.
First, a known Pickering emulsion does not have sufficient stability in an emulsified state. This stability indicates that a solid phase or a liquid phase and a liquid phase are not separated with the lapse of time.
More particularly, Non-Patent Literature 1 and Non-Patent Literature 2 merely create and study a simplified model containing spherical particles at an interface between a liquid phase (for example, an aqueous phase) and a liquid phase (for example, an oil phase) as a Pickering emulsion model, and do not pay attention to how the properties of a Pickering emulsion are influenced by the timing for adding inorganic particles in a relationship between an aqueous phase and a liquid phase and the shear speed and shear time for emulsification when a Pickering emulsion is formed. Therefore, the element and mechanism for controlling the stability of an emulsion are hardly clarified.

Particularly, when silica is adopted as inorganic particles, it is not mentioned how a hydrophobicity-hydrophilicity balance is influenced by a primary aggregate and a secondary aggregate formed due to the aggregating properties of silica.

In this regard, Non-Patent Literature 3 discloses an attempt to use hydrophobic silica as inorganic particles for adsorbing a hydrophilic polymer surfactant to the surface of the hydrophobic silica to adjust the hydrophobicity-hydrophilicity balance in order to change the properties of the emulsion.

However, Non-Patent Literature 3 has a problem in that a hydrophilic polymer surfactant is merely adsorbed using hydrophobic silica base as inorganic particles, and the type of an oil phase as a core is one, with the result that the type of oil agents to be emulsified is limited. In addition, even if the hydrophobicity-hydrophilicity balance influences the properties of an emulsion, the method for adjusting the hydrophobicity-hydrophilicity balance is complicated and expensive, and is not established as a product design technique in preparing an emulsion for the market.

Conventionally, such an industrial method for designing a Pickering emulsion with silica particles merely includes performing a pretreatment for the hydrophobization degree of silica at a raw material stage in order to partially hydrophobize silanol groups on the surface of silica for surrounding an oil by emulsification. Also, it is not clear what design parameter is to be noted for industrially producing a stabilized emulsion at a practical level.

Second, from the formed Pickering emulsion, a water content is removed to isolate and generate composite particle groups each containing an oil droplet as a core and silica particles existing on the surface (interface) of the oil droplet. In the composite particle group, the silica particles as inorganic particles coat the surface of an oil droplet such that the silica particles can firmly adhere to the core surface. The silica particles as a functional agent and the core as a different functional agent are advantageous when applied as a composite particle group to an application target, and thus expected to be applied as a composite particle in various uses, compared to when separately applied to an application target. In spite of such a fact, the problem of a variation among the composite particle groups is not studied.

More particularly, the specification of the composite particles includes a particle size, coating layer thickness, coating degree, and embedding degree (contact angle) of the silica particles to the core surface. The particle size, coating layer thickness, and coating degree are deeply involved in the hardness of the composite particles, and the embedding degree of the silica particles to the core surface is deeply associated with the adherence degree between the silica particles and the core. When such a specification varies among the composite particle groups, it is difficult to provide a reliable product.

Third, when a Pickering emulsion is formed, attention is not paid on adjusting the particle size, coating layer thickness, coating degree, and embedding degree (contact angle) as the specification of the composite particles depending on use applications.

More particularly, for example, in cosmetics uses, composite particles containing silicone rubber particles as a core are often employed. However, even if the particle size and coating layer thickness influence the sense during use, it is not examined what technique is used for adjusting the particle size and coating layer thickness.

More specifically, when the entire composite particle is soft, the sense of adherence to the skin after the composite particles have been applied is excellent. However, since the composite particles deform when applied, the contact area to the skin and the frictional resistance among particles increase. Accordingly, the extensibility of the composite particles on the skin decreases. Under such circumstances, there is a demand for a technology which softens rubber particles as a core and hardens a silica coating layer constituting an outer sheath, so that such extensibility is reliably achieved.

CITATION LIST

PATENT LITERATURE

    Patent Literature 1: Japanese Patent Application Laid-Open No. 2004-203735
    Patent Literature 2: Japanese Patent Application Laid-Open No. 2000-95632
    Patent Literature 3: Japanese Patent Application Laid-Open No. 2008-31487

NON-PATENT LITERATURE

Non-Patent Literature 1: J. Jpn. Soc. Colour Mater., 89(6), 203 (2016)
Non-Patent Literature 2: J. Chem. Phys., 124, 241104 (2006)
Non-Patent Literature 3: Master's thesis by Tomoyuki Suzuki, Department of Chemistry for Materials, Graduate School of Engineering (Master's Course), Mie University (2010)

SUMMARY OF INVENTION

TECHNICAL PROBLEM

In view of the above-described technical problem, an object of the present invention is to provide an aqueous dispersion which exhibits properties required of an aqueous dispersion and achieves the stability and homogeneity of an aqueous dispersion, and a method for producing the aqueous dispersion.

In view of the above-described technical problem, an object of the present invention is to provide an oil-in-water type emulsion which reliably achieves stability, and a method for producing the oil-in-water type emulsion.

In view of the above-described technical problem, an object of the present invention is to provide a method for designing the oil-in-water type emulsion which contributes to designing a practical emulsion product.

In view of the above-described technical problem, an object of the present invention is to provide composite particle groups isolated from the oil-in-water type emulsion, in which a variation among the composite particle groups is reduced.

In view of the above-described technical problem, an object of the present invention is to provide a method for producing composite particles which can adjust the specification of composite particles in the composite particle groups isolated from the oil-in-water type emulsion.

SOLUTION TO PROBLEM

The present inventors intensively conducted research. As a result, it was found that when a total molar ratio represented by (the total number of moles of surface hydrophilic groups of the entire inorganic particle groups obtained from each of the inorganic particle groups/the total number of moles of surface hydrophobic groups of the entire inorganic particle groups obtained from each of inorganic particle groups) is set to a prescribed lower limit value or more and/or a prescribed upper limit value or lower depending on the aggregating properties of inorganic particles, it is determined which of a self-micelle-like aggregate, a hydrophobic-rich aggregate, and a hydrophilic-rich aggregate is voluntarily generated. Also, it was found that an aqueous dispersion according to its purpose such as imparting thickening properties and thixotropy can be provided. Furthermore, it was found that a self-micelle-like aggregate is particularly excellent in stability and homogeneity.

Furthermore, it was found that when the inorganic particles are fumed silica, an aqueous dispersion being rich in self-micelle-like aggregates can be generated by setting the above-described total mole number ratio to fall within the range of 20/80 to 80/20 at a raw material silica stage.

Also, it was found that when an oil-in-water type emulsion (Pickering emulsion) to be obtained by coating an oil droplet with a self-micelle-like aqueous dispersion of fumed silica particles is mainly applied with shear force at a speed of a prescribed value or more at an aqueous dispersion stage, the rearrangement of primary aggregates among secondary aggregates and/or the orientation of primary aggregates inside secondary aggregates are promoted. Accordingly, a variation of the hydrophobization degree among composite particles can be reduced, and a more stable and homogeneous oil-in-water type emulsion is generated.

Also, as a method for checking the degree of the above-described variation in a simple manner, a method of utilizing the measurement result of a thixotropy index at an aqueous dispersion stage was found.

Also, a designing method for obtaining a stable and homogeneous oil-in-water type emulsion of fumed silica particles was found.

Furthermore, a method for producing the composite particles, which can adjust the specification of the composite particles in the composite particle groups isolated from the oil-in-water type emulsion, was found.

The present invention has been accomplished on the basis of such knowledge.

That is, the aqueous dispersion and the method for producing the aqueous dispersion, as well as the oil-in-water type emulsion and the method for producing and designing the oil-in-water type emulsion according to the present invention are as follows.

[1] An aqueous dispersion of inorganic particles, including inorganic particle groups dispersed in an aggregation state in water, wherein a total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is within a value range of a prescribed lower limit value or more depending on the aggregating properties of inorganic particles, whereby the inorganic particle groups contain self-micelle-like aggregates and hydrophobic-rich aggregates.

[2] An aqueous dispersion of inorganic particles, including inorganic particle groups dispersed in an aggregation state in water, wherein a total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is within a value range of a prescribed upper limit value or less depending on the aggregating properties of inorganic particles, whereby the inorganic particle groups contain self-micelle-like aggregates and hydrophilic-rich aggregates.

[3] An aqueous dispersion of inorganic particles, including inorganic particle groups dispersed in an aggregation state in water, wherein a total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is within a value range of a prescribed lower limit value or more and a prescribed upper limit value or less depending on the aggregating properties of inorganic particles, whereby the inorganic particle groups contain self-micelle-like aggregates.

[4] The aqueous dispersion of inorganic particles according to any one of [1] to [3], wherein the inorganic particle groups contain silica.

[5] The aqueous dispersion of inorganic particles according to any one of [1] to [3], wherein the inorganic particle groups contain fumed silica, the prescribed lower limit value is 20/80, and the prescribed upper limit value is 80/20.

[6] A method for producing an aqueous dispersion of fumed silica particles, including fumed silica particle groups dispersed in an aggregation state in water, wherein a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 20/80 or more at a raw material stage of the fumed silica particle groups, and the aqueous dispersion of the fumed silica particles contains self-micelle-like aggregates and hydrophobic-rich aggregates.

[7] A method for producing an aqueous dispersion of fumed silica particles, including fumed silica particle groups dispersed in an aggregation state in water, wherein a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 80/20 or more at a raw material stage of the fumed silica particle groups, and the aqueous dispersion of the fumed silica particle contains self-micelle-like aggregates and hydrophilic-rich aggregates.

[8] A method for producing an aqueous dispersion of fumed silica particles, including fumed silica particle groups dispersed in an aggregation state in water, wherein a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 20/80 or more and 80/20 or less at a raw material stage of the fumed silica particle groups, and the aqueous dispersion of the fumed silica particle contains self-micelle-like aggregates.

[9] A method for producing an aqueous dispersion containing a self-micelle-like aggregate group by fumed silica particle groups at a secondary aggregation level, including

a stage of preparing fumed silica particle groups at a secondary aggregation level such that a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is 20/80 or more and 80/20 or less, and

a stage of adding the prepared fumed silica particle groups at the secondary aggregation level to water to form self-micelle-like aggregates of the fumed silica particle groups at a secondary aggregation level.

[10] The method for producing an aqueous dispersion of fumed silica particles according to any one of [8] and [9], including a process of applying a shear force that is sufficient for causing the exchange of the fumed silica particle groups at a primary aggregation level among the fumed silica particle groups at the secondary aggregation level while the fumed silica particle groups are dispersed in water.

[11] An oil-in-water type emulsion, including composite particle groups each having an oil contained in self-micelle-like aggregates of fumed silica particle groups at a secondary aggregation level in which a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is 20/80 or more and 80/20 or less, wherein

each of the composite particle groups contain an oil droplet coated with the self-micelle-like aggregates.

[12] The oil-in-water type emulsion according to [11], wherein a difference between an average value of the total mole number ratio and a molar ratio represented by (the number of moles of surface hydrophilic groups/the number of moles of surface hydrophobic groups) in each of the fumed silica particle groups is a prescribed value or less, whereby homogenization among the self-micelle-like aggregates is achieved.

[13] The oil-in-water type emulsion according to any one of [11] and [12], wherein the oil is a curable rubber composition or a rubber-containing oil which maintains fluidity.

[14] A method for producing an oil-in-water type emulsion, including:

a stage of preparing fumed silica particle groups at a secondary aggregation level such that a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) becomes 20/80 or more and 80/20 or less;

a stage of adding the prepared fumed silica particle groups at the secondary aggregation level to water and applying a shear force at a prescribed shear speed to promote the exchange at a primary aggregation level among the fumed silica particle groups at the secondary aggregation level and/or the orientation at the primary aggregation level in the fumed silica particles at the secondary aggregation level, thereby generating an aqueous dispersion containing self-micelle-like aggregates of the fumed silica particle groups at the secondary aggregation level such that a difference between an average value of the total mole number ratio and a molar ratio represented by (the number of moles of surface hydrophilic groups/the number of moles of surface hydrophobic groups) in each of the secondary particles constituting the fumed silica particle groups becomes a prescribed value or less; and

a stage of adding an oil to the generated aqueous dispersion to form an emulsion, whereby the oil-in-water type emulsion contains composite particle groups including the oil inside the self-micelle-like aggregates of the fumed silica particle groups at the secondary aggregation level.

[15] The method for producing an oil-in-water type emulsion according to [14], wherein the stage of generating an aqueous dispersion containing self-micelle-like aggregates of fumed silica particle groups includes a stage of creating master thixotropy index curves by plotting thixotropy indices of two master aqueous dispersions which have the molar ratio variation close to 0 and a maximum value with respect to a total mole number ratio while previously knowing a molar ratio variation that is a difference between an average value of the total mole number ratio and a molar ratio represented by (the number of moles of surface hydrophilic groups/the number of moles of surface hydrophobic groups) in each of the same type of fumed silica particle groups, measuring a thixotropy index of an aqueous dispersion prepared at an optional total mole number ratio, comparing the measured thixotropy index to the master thixotropy index curves at the total molar ratio to calculate the molar ratio variation of the prepared aqueous dispersion by proportional distribution, and setting the molar ratio variation to a prescribed value or less.

[16] The method for producing an oil-in-water type emulsion according to any one of [14] and [15], wherein the stage of forming an emulsion further includes applying an auxiliary shear force at a prescribed shear speed to promote the orientation at a primary aggregation level in the fumed silica particle groups at a secondary aggregation level.

[17] The method for producing an oil-in-water type emulsion according to any one of [14] to [16], further including adjusting a mass ratio between the oil and the fumed silica particle groups to adjust a coating degree and/or a coating thickness of the self-micelle-like aggregates on the surface of an oil droplet in the composite particle groups.

[18] A method for designing an oil-in-water type emulsion aiming at achieving stability of an emulsion, including:

a stage of obtaining a master thixotropy index curve of a master aqueous dispersion including fumed silica particle groups dispersed in an aggregation state in water, in which a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) and a molar ratio variation that is a difference between an average value of the total mole number ratio represented by (the number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) and a molar ratio represented by (the number of moles of surface hydrophilic groups/the number of moles of surface hydrophobic groups) in each of the fumed silica particle groups are previously known;

a stage of measuring a thixotropy index of an aqueous dispersion including fumed silica particle groups of the same type as and the same total mole number ratio as the master aqueous dispersion dispersed in water in an aggregation state;

a stage of comparing the measured thixotropy index with the master thixotropy index curve to evaluate the molar ratio variation of the aqueous dispersion; and

a stage of adding an oil to the aqueous dispersion having been evaluated for the molar ratio variation, to generate an oil-in-water type emulsion containing composite particle groups containing the oil inside self-micelle-like aggregates of the fumed silica particle groups at a secondary aggregation level.

[19] The method for designing an oil-in-water type emulsion according to [18], wherein the stage of evaluating the molar ratio variation of an aqueous dispersion includes creating master thixotropy index curves by plotting thixotropy indices of two master aqueous dispersions which have the molar ratio variation close to 0 and a maximum value with respect to the total mole number ratio while previously knowing a molar ratio variation that is a difference between an average value of the total mole number ratio and a molar ratio represented by (the number of moles of surface hydrophilic groups/the number of moles of surface hydrophobic groups) in each of the same type of fumed silica particle groups, measuring a thixotropy index of an aqueous dispersion prepared at an optional total mole number ratio, comparing the measured thixotropy index with the master thixotropy index curves at the total molar ratio, and calculating the molar ratio variation of the prepared aqueous dispersion by proportional distribution.

[20] The method for designing an oil-in-water type emulsion according to any one of [18] and [19], including previously grasping the molar ratio variation of the master aqueous dispersion by carbon analysis and oxygen analysis of the composite particle groups through an electron microscope.

ACTION OF INVENTION

According to the aqueous dispersion of inorganic particles having the above-described configuration, stability as well as high viscosity and thixotropy properties can be expressed. The reason for this is as follows. The higher the hydrophobic degree of the surface of inorganic particles, the more likely the particles aggregate, and the more likely the hydrophobic-rich aggregates are generated. Also, the higher the hydrophilicity of the surface, the less likely the particles aggregate. Therefore, when a total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is within a value range of a prescribed lower limit value or more, hydrophilic-rich aggregates, which can be generated under the condition of the above-described prescribed lower limit value or less, are not generated. Accordingly, the inorganic particle groups come to contain self-micelle-like aggregates and hydrophobic-rich aggregates.

According to the aqueous dispersion of inorganic particles having the above-described configuration, stability as well as low viscosity and high solubility can be expressed. The reason for this is as follows. The higher the hydrophobic degree of the surface of inorganic particles, the more likely the particles aggregate, and the more likely the hydrophobic-rich aggregates are generated. Also, the higher the hydrophilicity of the surface, the less likely the particles aggregate. Therefore, when a total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is set so as to fall within a value range of a prescribed upper limit value or less, hydrophobic-rich aggregates, which can be generated under the condition of the prescribed upper limit value or more, are not generated. Accordingly, the inorganic particle groups come to contain self-micelle-like aggregates and hydrophilic-rich aggregates.

According to the aqueous dispersion of inorganic particles having the above-described configuration, both stability and homogeneity are achieved. In addition, moderate viscosity, thixotropy, and high solubility can be expressed. The reason for this is as follows. The higher the hydrophobic degree of the surface of inorganic particles, the more likely the particles aggregate, and the more likely the hydrophobic-rich aggregates are generated. Also, the higher the hydrophilicity of the surface, the less likely the particles aggregate. Therefore, when a total mole number ratio represented by (the total number of moles of surface hydrophilic groups of the entire inorganic particle groups obtained from each of the inorganic particle groups/the total number of moles of surface hydrophobic groups of the entire inorganic particle groups obtained from each of the inorganic particle groups) is set so as to fall within a value range of a prescribed lower limit value or more and a prescribed upper limit value or less, hydrophilic-rich aggregates, which can be generated under the condition of a prescribed lower limit value or less, are not generated, and hydrophobic-rich aggregates, which can be generated under the condition of a prescribed upper limit value or more, are not generated. Accordingly, the inorganic particle groups come to contain self-micelle-like aggregates.

According to the method for producing an aqueous dispersion of fumed silica particles having the above-described configuration, stability as well as high viscosity and thixotropy can be promoted. The reason for this is as follows. When a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 20/80 or more at a raw material stage of the fumed silica particle groups, hydrophilic-rich aggregates, which are generated when the total mole number ratio is 20/80 or less, are not generated. Accordingly, self-micelle-like aggregates and hydrophobic-rich aggregates come to be mainly contained.

According to the method for producing an aqueous dispersion of fumed silica particles having the above-described configuration, stability as well as low viscosity and high solubility can be promoted. The reason for this is as follows. When a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 20/80 or less at a raw material stage of the fumed silica particle groups, hydrophobic-rich aggregates, which are generated when the total mole number ratio is 80/20 or more, are not generated. Accordingly, self-micelle-like aggregates and hydrophilic-rich aggregates come to be mainly contained.

According to the method for producing an aqueous dispersion of fumed silica particles having the above-described configuration, stability as well as low viscosity and high solubility can be promoted. The reason for this is as follows. When a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 20/80 or more and 80/20 or less at a raw material stage of the fumed silica particle groups, hydrophilic-rich aggregates, which are generated when the total mole number ratio is 20/80 or less, are not generated, and hydrophobic-rich aggregates, which are generated under the condition of 80/20 or more, are not generated. Accordingly, self-micelle-like aggregates come to be mainly contained. Both stability and homogeneity are achieved, and moderate viscosity, thixotropy, and high solubility can be promoted.

The method for producing the aqueous dispersion having the above-described configuration includes preparing fumed silica particle groups at a secondary aggregation level such that a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) becomes 20/80 or more and 80/20 or less, and adding the prepared fumed silica particle groups at a secondary aggregation level to water and applying a shear force at a prescribed shear speed. Accordingly, when the total mole number ratio is 20/80 or less, the fumed silica particle groups at a secondary aggregation level in the aqueous dispersion come to mainly contain hydrophilic-rich aggregates. Therefore, stability as an aqueous dispersion can be achieved, but the ratio of self-micelle-like aggregates is low. On the other hand, when the total mole number ratio is 80/20 or more, hydrophobic-rich aggregates come to be mainly contained. Therefore, stability as an aqueous dispersion is not achieved, and the ratio of self-micelle-like aggregates is low. However, when it is 20/80 or more and 80/20 or less, generation of self-micelle-like aggregates can be promoted.

According to the oil-in-water type emulsion having the above-described configuration, it is possible to promote the stability and homogeneity of the composite particle groups containing an oil droplet coated with the fumed silica particle groups having the structure of self-micelle-like aggregates. The reason for this is as follows. When a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is set so as to become 20/80 or more and 80/20 or less, since stable and homogeneous self-micelle-like aggregates come to be mainly contained, an oil can be contained inside the aggregates in such a state.

The method for producing the oil-in-water type emulsion having the above-described configuration includes preparing fumed silica particle groups at a secondary aggregation level such that a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) becomes 20/80 or more and 80/20 or less, and adding the prepared fumed silica particle groups at a secondary aggregation level to water and applying a shear force at a prescribed shear speed. Accordingly, when the total mole number ratio is 20/80 or less, the fumed silica particle groups at a secondary aggregation level in the aqueous dispersion come to mainly become hydrophilic-rich aggregates. Therefore, stability as an aqueous dispersion can be reliably achieved, but the ratio of self-micelle-like aggregates is low. On the other hand, when the total mole number ratio is 80/20 or more, hydrophobic-rich aggregates come to be mainly contained. Therefore, stability as an aqueous dispersion is not achieved, and the ratio of self-micelle-like aggregates is low. However, when it is 20/80 or more and 80/20 or less, and a variation between an average value of the total mole number ratio and a molar ratio represented by (the number of moles of surface hydrophilic groups/the number of moles of surface hydrophobic groups) in each of the fumed silica particle groups is a prescribed value or less, the exchange at a primary aggregation level among the fumed silica particle groups at a secondary aggregation level and/or the orientation at a primary aggregation level in the fumed silica particles at a secondary aggregation level can be promoted on the basis of the aggregating properties of the fumed silica particles. Accordingly, self-micelle-like aggregates with a hydrophilic portion outside at a high density and a hydrophobic portion inside at a high density come to be mainly contained. This can promote the homogenization among the mainly contained self-micelle-like aggregates.

When a shear force is applied at a prescribed shear speed or less during the generation of an aqueous dispersion, the exchange at a primary aggregation level among the fumed silica particle groups at a secondary aggregation level and/or the orientation at a primary aggregation level in the fumed silica particles at a secondary aggregation level are not promoted.

Subsequently, an oil is added to the generated aqueous dispersion to form an emulsion, so that the oil is contained in a hydrophobic portion located inside the self-micelle-like aggregates. The emulsion comes to contain composite particle groups each having the oil contained inside self-micelle-like aggregates formed of fumed silica particle groups at a secondary aggregation level. Such an emulsion can reliably maintain stability over the time course.

The method for designing the oil-in-water type emulsion having the above-described configuration includes:; obtaining master thixotropy index curves of the master aqueous dispersions of which a total mole number ratio between surface hydrophilic groups and surface hydrophobic groups and a molar ratio variation that is a difference between an average value of the total mole number ratio and a molar ratio represented by (the number of moles of surface hydrophilic groups/the number of moles of surface hydrophobic groups) in each of the fumed silica particle groups) for each of master aqueous dispersions including fumed silica particle groups dispersed in water in an aggregation state are previously known, measuring a thixotropy index of an aqueous dispersion including fumed silica particle groups of the same type as and the same total mole number ratio as the master aqueous dispersion in an aggregation state in water; and comparing the measured thixotropy index with the master thixotropy index curves to evaluate the molar ratio variation of the aqueous dispersion. Thus, the variation value of the prepared aqueous dispersion can be grasped. That is, the existence ratio of self-micelle-like aggregates can be grasped further precisely, quantitatively, and simply.

At a subsequent stage, an oil is added to the aqueous dispersion having been evaluated for the molar ratio variation. Accordingly, the stage includes generating an oil-in-water type emulsion containing composite particle groups having the oil inside self-micelle-like aggregates formed of fumed silica particle groups at a secondary aggregation level. Therefore, an oil-in-water type emulsion is prepared after a certain ratio or more of self-micelle-like aggregates has been generated in an aqueous dispersion depending on its intended use at the previous stage. Therefore, an oil-in-water type emulsion being excellent in stability and homogeneity can be designed further precisely and reliably.

Advantageous Effects of Invention

According to the present invention, by changing the total molar ratio represented by (the number of moles of the surface hydrophilic group/the number of moles of the surface hydrophobic group) depending on the aggregating properties of the inorganic particles, it is possible to change the generation ratios of the self-micelle-like aggregates, the hydrophobic-rich aggregates, and the hydrophilic-rich aggregates as the type of the aqueous dispersion. As a result, an aqueous dispersion of inorganic particles having performances according to purposes such as thickening properties and thixotropy can be obtained.

According to the present invention, a stable and homogeneous aqueous dispersion of fumed silica being rich in self-micelle-like aggregates can be obtained with a clear and simple setting at the raw material silica stage. According to the present invention, an oil-in-water type emulsion (Pickering emulsion) in which a stable and homogeneous self-micellar aqueous dispersion of fumed silica particles is coated on oil droplets can be produced by promoting the exchange and/or orientation of the silica particles at the stage of the aqueous dispersion.

According to the present invention, since the thixotropy index can be used at the stage of the aqueous dispersion as a method of simply checking the degree of the variation, an oil-in-water type emulsion (Pickering emulsion) with very high reliability can be produced. The present invention provides a design for obtaining a stable and homogeneous oil-in-water type emulsion of fumed silica particles.

According to the present invention, since the specification of the composite particles in the oil-in-water type emulsion can be adjusted, stable, homogeneous and highly reliable composite particles can be isolated.

DESCRIPTION OF EMBODIMENTS

Although it has been known that the balance between hydrophilicity and hydrophobicity of solid particles existing at the interface between an aqueous phase and an oil phase is an important factor for the emulsion stability in an oil-in-water type Pickering emulsion using solid particles as compared to an oil-in-water type emulsion using a surfactant, the inventors have found that it is important for the stability of an emulsion to form an oil-in-water Pickering emulsion by adding an oil to the solid particles, particularly in the case of silica particles, based on an aqueous dispersion in which silica particles are dispersed in water, and on the basis of this fact, also found that the formation mechanism of a composite particle structure of silica particles and oil in a Pickering emulsion is unique due to the aggregation of silica particles. The inventors have thus paid attention to not only the stability of the oil-in-water type Pickering emulsion, but also, as a parameter for evaluating the stability of the aqueous dispersion in which silica particles are dispersed in water, variations in so-called total mole number ratio and total molar ratio of the surface hydrophilic group and the surface hydrophobic group of the silica particles, and found that it is possible to relax restrictions on the selection of the silica particles and the oil component as the starting raw materials, and also that it is possible to balance the stability and properties of the aqueous dispersion according to the use application, and to adjust the composite particle structure in the emulsion according to the use application.

Examples of the inorganic particles used as the raw material for the aqueous dispersion of the inorganic particles of the present invention may include inorganic particles such as silica, titanium dioxide, zinc oxide, talc, kaolin, mica, vermiculite, magnesium carbonate, calcium carbonate, aluminum silicate, barium silicate, calcium silicate, magnesium silicate, strontium silicate, a tungstic acid metal salt, magnesium, zeolite, barium sulfate, calcined calcium sulfate, calcium phosphate, fluorine apatite, hydroxyapatite, and metal soap. Furthermore, a composite particle obtained by coating a metal oxide or the like onto a particle, or a modified particle in which a surface of the particle is treated with a compound or the like may be used.

Usually, the surface of these particles includes a portion that is covered with a hydrophilic group such as a silanol group, a carbinol group, or another hydroxyl group, and a portion that is covered with a group obtained by hydrophobizing these groups with an alkyl group or the like, or another hydrophobic group. By adjusting the ratio of the hydrophilic groups to the hydrophobic groups, the stability and homogeneity of the aqueous dispersion can be controlled by the present invention with respect to the aggregating properties, solubility, and the like of the inorganic particles by the present invention, and the aggregating properties and solubility of the particles can be controlled by the balance between the hydrophilicity and the hydrophobicity.

Among the inorganic particles, silica is preferably used. Examples of the type of silica may include fumed silica, wet silica, and colloidal silica. Since silanol which is hydrophilic exists on the surface in any type of silica particle and the silanol group can be subjected to a hydrophobic treatment by an alkyl group or the like at any ratio, it is easy to set the molar ratio of the hydrophilic groups and the hydrophobic groups on the surface thereof. The silica is preferable because it forms an aggregate structure, has a high affinity with various oils and thus is easily transferred to a Pickering emulsion, and provides a wide range of use applications from the viewpoints of availability, economy, and the like.

A ratio of the silica particles in the inorganic particles does not always need to be 100%, and the silica particles may be partially contained in the inorganic particles. This is because the silica particles form a network with the inorganic particles other than the silica particles, and play a leading role in stabilizing and homogenizing the aqueous dispersion while the other inorganic particles can auxiliary participate in such a role.

The most preferable silica as the raw material of the aqueous dispersion is a fumed silica.

The fumed silica particles form a multidimensional aggregate structure. Thus, it is possible to control the balance between the surface hydrophilic groups and hydrophobic groups and rearrange aggregation units according to the aggregate level. This allows the most effective control of the stability and homogeneity of the aqueous dispersion for implementation of the present invention.

Further, the fumed silica particle, which has a porous structure, has a large surface area and more enhanced functions in association and adsorption, making it possible to produce the more stable and homogenous aqueous dispersion. Further, the increased functions of associating and adsorbing to various oils provide an advantage in transferring to the Pickering emulsion.

The primary particle of the fumed silica particle as a minimum unit normally has a size of about 5 to 30 nanometers. The primary particles are aggregated each other to form the primary aggregate, that is, the secondary particle. The primary aggregate normally has a size of about 100 to 400 nanometers. The primary particles are fused together by a chemical bond, and thus it is normally difficult to disassemble the primary aggregate. Further, the primary aggregates are aggregated each other to form an aggregate structure, which is referred to as a secondary aggregate or a tertiary particle. The secondary aggregate has a size of about 10 $\mu$m. An aggregate form between the primary aggregates in the secondary aggregate is not normally caused by the chemical bond, but by an associative aggregation force such as a hydrogen bond. Thus, a part or the whole of the secondary aggregate can be disassembled to the primary aggregate level by applying some external force, for example, by applying a shearing force at a predetermined level or more in water. The disassembled secondary aggregate can be reaggregated into the original secondary aggregate by removing the external force.

Note that, in the description of the present invention, the primary and secondary aggregates are also appropriately referred to as the silica particle groups at the primary and secondary aggregate levels, respectively.

For the fumed silica particle in a powder state, the secondary aggregate is usually the largest aggregation level. However, the secondary aggregates can be further aggregated each other in the aqueous dispersion. Such an aggregate can be disassembled by a force weaker than the one used for disassembling the secondary aggregate.

However, the above-described aggregate levels such as the primary aggregate and the secondary aggregate are not always formed in a clearly distinct manner. For example, the aggregate levels at the intermediate stages between the primary aggregate and the secondary aggregate may be continuously distributed in some degree and coexist. The primary aggregate and the secondary aggregate are defined by including such a distribution range.

Incidentally, the fumed silica particles are also known to form a network structure, particularly at the secondary aggregate level, which may be useful as inorganic particles for Pickering emulsions.

Hereinafter, the state in water of the aqueous dispersion of inorganic particles according to the present invention, the method for producing the aqueous dispersion, and the use application of the aqueous dispersion, when a fumed silica is used as a raw material, will be described. In the description, the term "primary aggregation level" or "secondary aggregation level" not only indicates 100% of the level, but also indicates being substantially at the aggregation level.

Even when an aqueous dispersion contains inorganic particles other than fumed silica, the basic part is the same.

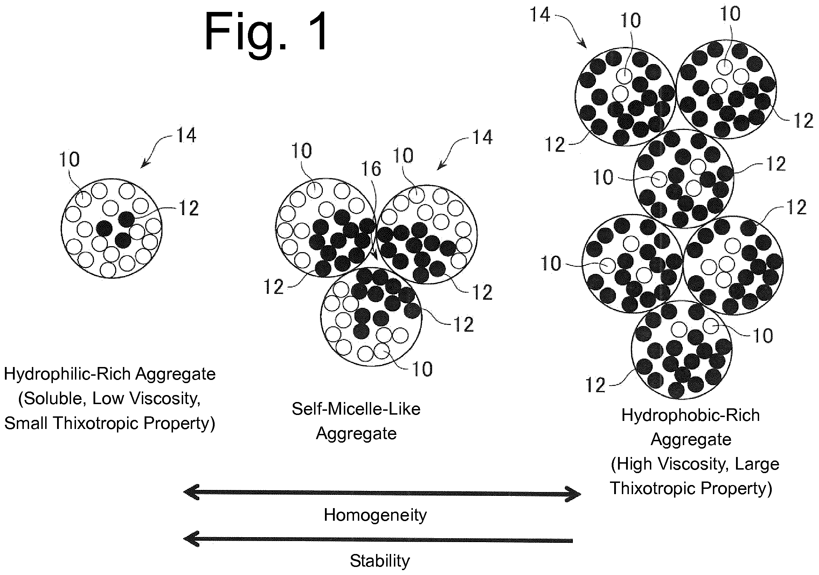

Fig. 1 illustrates what aggregates can be generated when fumed silica particles are dispersed in water. As described herein, the terms "hydrophilic-rich aggregate", "hydrophobic-rich aggregate", and "self-micelle-like aggregate" are used for descriptive purposes. The self-micelle-like aggregate is a new concept found in the present invention. Also, since the relationship among these three aggregates has been found in the present invention, it will be described.

When the molar ratio of (hydrophilic groups/hydrophobic groups) on the surface of fumed silica particles is hydrophilic group-rich, the silica particle surface has an affinity to water. Therefore, silica particles are likely to dissolve in water, and a secondary aggregate 14 does not aggregate any further and often singly dissolves in water in a stable manner. The viscosity of the aqueous dispersion is low, and the thixotropy is low. Such an aggregate is referred to as a hydrophilic-rich aggregate.

When the molar ratio of (hydrophilic groups/hydrophobic groups) on the surface of fumed silica particles is hydrophobic group-rich, the silica particle surface has a less affinity to water. Therefore, silica particles tend to be arranged toward a direction in which a contact area between silica particles and water is as small as possible. This facilitates the aggregation of silica particles. A plurality of secondary aggregates 14 gather in water to form an aggregate. This increases the viscosity of the aqueous dispersion and enhances thixotropy. In this state, the stability of the aqueous dispersion is not favorable. Silica particles are likely to, for example, settle down with the lapse of time. Such an aggregate is referred to as a hydrophobic-rich aggregate. This state already has proposed its use applications as a thickening agent and a thixotropy imparting agent as hydrophobic fumed silica. However, there is the restriction that a user needs to disperse silica particles into water and immediately pour the dispersion into an intended material.

When the molar ratio of (hydrophilic groups/hydrophobic groups) on the surface of fumed silica particles is in a region between a hydrophilic-rich aggregate and a hydrophobic-rich aggregate, a certain aggregate is formed in the secondary aggregate 14. The formed aggregate has a portion containing a relatively high-hydrophilic primary aggregate 10 at a high density, which is in contact with an aqueous phase, and a portion containing a relatively high-hydrophobic primary aggregate 12 at a high density, which is in contact with another secondary particle. Since this aggregate has a similar aggregation form to a self-micelle of a common surfactant, it is referred to as a self-micelle-like aggregate. In this state, the number of aggregates in the secondary aggregate 14 is small, and the size of the aggregates spontaneously becomes homogeneous. Therefore, a stable and homogeneous aqueous dispersion is obtained. Since the concentration of hydrophilic groups is high outside the self-micelle-like aggregate, that is, high in a portion which is in contact with an aqueous phase, sufficiently high stability comparable to that of the hydrophilic-rich aggregate can be obtained. The stability of the self-micelle-like aggregate is considerably higher than that of the hydrophobic-rich aggregate. Since a space 16 located inside the self-micelle-like aggregate has a lipophilic environment, an oil is likely to be incorporated from the outside. The viscosity and thixotropy of the self-micelle-like aggregate lie halfway between the hydrophilic-rich aggregate and the hydrophobic-rich aggregate. A self-micelle of a surfactant is formed when a surfactant becomes excessive in relation to an oil to be emulsified. However, fumed silica particles can be formed independently from the existence of an oil with less influence by concentration.

At a low concentration, a surfactant sufficiently interacts with a water molecule and dissolves into a surfactant-molecule or surfactant-ion state (in a monodispersion state). However, at a specific concentration or more, the concentration of the monodispersion state is saturated, and the surfactant molecules after the saturation associate with each other to form a supermolecule or a self-organism, which is a so-called self-micelle. A self-micelle usually has an association structure with a hydrophilic portion outside and a hydrophobic portion inside.

The present inventors have paid attention to the fact that at more than a saturation concentration, a self-micelle-like aggregate having such an association structure with a hydrophilic portion outside and a hydrophobic portion inside is similarly formed in a state in which silica at a secondary aggregation level is dispersed in water. However, the saturation concentration is considerably lower than when a surfactant is used.

The relative definitions of these three types of aggregates are as follows.

The hydrophobic-rich aggregate as an aggregate of fumed silica particles is a lump of fumed silica particle groups which has a high aggregation level and exhibits hydrophobicity to such a degree as to express thixotropy, compared to the self-micelle-like aggregate and the hydrophilic-rich aggregate. The hydrophilic-rich aggregate as an aggregate of fumed silica particles is a lump of fumed silica particle groups which has a low aggregation level and exhibits hydrophobicity to such a degree as to dissolve in water, compared to the self-micelle-like aggregate and the hydrophobic-rich aggregate. The self-micelle-like aggregate as an aggregate of fumed silica particles is a lump of fumed silica particle groups which has an aggregation level lying between the hydrophobic-rich aggregate and the hydrophilic-rich aggregate and exhibits hydrophobicity inside and hydrophilicity outside, compared to the hydrophobic-rich aggregate and the hydrophilic-rich aggregate. Furthermore, the self-micelle-like aggregate has a structure that includes a lipophilic space inside and is likely to incorporate an oil from the outside.

Some inorganic particles other than fumed silica particles have such three types of aggregation forms.

When the aqueous dispersion including the fumed silica particles is produced, regardless of the types of the aggregates to be formed, the silica particles as the raw material are hardly spontaneously dissolved or dispersed in water, and thus it is necessary to use some device which applies a shearing force. The production can be performed by using a conventional mixer, for example, a homogenizer, a colloid mill, a homomixer, and a high-speed stator-rotor stirring device.

The compounding amount of the fumed silica particles in the aqueous dispersion is preferably within a range of 0.1 to 30.0 mass%, more preferably within a range of 0.2 to 10 mass%, relative to the total amount of the aqueous dispersion composition. If the compounding amount is less than 0.1 mass%, the aggregation sometimes does not proceed, while if the compounding amount is more than 30.0 mass%, the viscosity becomes too large and exceeds a proper range of the stirring device.

The aggregation force between the primary aggregates of the fumed silica particles is released by applying a specific shearing force, and the primary aggregates are aggregated again when the shearing is removed. Thus, in the production of aqueous dispersions, the primary aggregates may be exchanged between separate secondary aggregates upon application of a specific shear. This makes it possible to change a ratio between the hydrophilic-rich primary aggregates and the hydrophobic-rich primary aggregates in the single secondary aggregate. Such an exchange of the primary aggregates between the secondary aggregates is referred to as rearrangement.

The rearrangement is caused preferably by applying shearing at a speed of 7,500 s$^{-1}$ or more. The shearing speed less than 7,500 s$^{-1}$ does not cause the sufficient rearrangement. The upper limit value of the shearing speed for causing the rearrangement is not limited; however, if the shearing speed is more than 100,000 s$^{-1}$, for example, the following problems may arise: a malfunction of the device tends to occur, a water content of the aqueous dispersion is evaporated by heating, and the oil, if present, is decomposed. Thus, it is not preferable.

Causing the sufficient rearrangement promotes the exchange of the primary aggregates between the secondary aggregates. As a result, the ratio between the hydrophilic-rich primary aggregates and the hydrophobic-rich primary aggregates in the secondary aggregate of the fumed silica particles becomes more homogenous in the entire aqueous dispersion system.

Also, if a shearing speed enough for causing the rearrangement to occur is applied to the aggregates of silica particles, movement of the primary aggregates within the same secondary aggregates may occur and the arrangement may change. Further, when the self-micelle-like aggregate is formed, as shown in FIG. 1, the hydrophobic primary aggregates may be oriented toward an aggregation direction of two particles.

As described above and schematically shown in FIG. 1, the types of the most easily formed aggregates change depending on a degree of hydrophilicity/hydrophobicity of the fumed silica particles.

More specifically, if the total mole number ratio represented by (the total number of moles of surface hydrophilic groups of the entire fumed silica particle groups obtained from each of the fumed silica particle groups /the total number of moles of the surface hydrophobic groups of the entire fumed silica particle groups obtained from each of the fumed silica particle groups) is a predetermined lower limit or less, the hydrophobic-rich aggregates are primarily produced. Further, if the total molar ratio is a predetermined upper limit or more, the hydrophilic-rich aggregates are primarily produced. Further, if the total molar ratio is the predetermined lower limit or more and the predetermined upper limit or less, the self-micelle-like aggregates are primarily produced.

In case of the fumed silica, the lower limit value of the total molar ratio is about 20/80. The upper limit value is about 80/20.

In the present invention, it was unexpectedly found that applying a certain level or more of shearing force causes the rearrangement of the fumed silica particles without being restricted by the state of the fumed silica as a starting material. That is, at a stage of a raw material, the total mole number ratio represented by (the total number of moles of

the surface hydrophilic groups of the entire fumed silica particle groups obtained from each of the fumed silica particle groups/the total number of moles of the surface hydrophobic groups of the entire fumed silica particle groups obtained from each of the fumed silica particle groups) is only required to be set to 20/80 or more and/or 80/20 or less for any types of the silica as a raw material. The hydrophilic silica particle in which a surface silanol group is remained, the hydrophobic silica particle having a surface silanol group having been subjected to the hydrophobic treatment, or a mixture thereof at any ratio may be used. Further, the fumed silica having been subjected to the hydrophobic treatment at any ratio may be used. Commercially available fumed silica particles can be used. Alternatively, the hydrophilic silica particle can be hydrophobized by a known method of treating the hydrophilic silica particle with halogenated organic silicon such as methyltrichlorosilane, alkoxysilanes such as dimethyldialkoxysilane, silazane, and low molecular weight methylpolysiloxane.

The specific surface area under a dry condition of the silica particle described above is preferably 2 to 350 $m^2$/g in a fine powder state, particularly preferably 50 to 300 $m^2$/g.

As a raw material, the above-described types of fumed silica particles can be used; however, at the stage of a raw material, the total mole number ratio represented by (the total number of moles of the surface hydrophilic groups of the entire fumed silica particle groups obtained from each of the fumed silica particle groups/the total number of moles of the surface hydrophobic groups of the entire fumed silica particle groups obtained from each of the fumed silica particle groups needs to be set to 20/80 or more and/or 80/20 or less.

The aqueous dispersion including the inorganic particles obtained by the present invention is stable and homogenous and has a function of imparting the thickening property and the thixotropic property. Such an aqueous dispersion has not been available up to now, thus a very large number of new use applications can be developed. Further, the aqueous dispersion of the present invention can be produced by adjusting the molar ratio of the hydrophilic groups and the hydrophobic groups at the stage of a raw material using a commercially available silica and a conventional device, thereby offering great advantages in costs and processes.

According to the present invention, the existence ratio of the hydrophobic-rich aggregates, the hydrophilic-rich aggregates, or the self-micelle-like aggregates can be changed by setting the total molar ratio of the fumed silica particles. This allows development of new use applications.

The self-micelle-like aggregate has functions of imparting the thickening effect and the thixotropic property, though not so much as the hydrophobic-rich aggregate. Further, the self-micelle-like aggregate is excellent in stability and homogeneity.

Thus, setting the total molar ratio to 20/80 or more causes a state in which the self-micelle-like aggregates and the hydrophobic-rich aggregates coexist. In such a case, the stability is increased compared with a thickening agent and a thixotropy imparting agent using the conventional hydrophobic fumed silica. That is, new use applications with the emphasis on both the stability and the thickening/the thixotropic properties can be developed. For example, when the aqueous dispersion is used in a coating material and a polymer material for imparting the thickening property and the thixotropic property, instability of easily causing precipitation of the silica particles conventionally found is not exhibited, and thus the commercial production can be possible as the stable aqueous dispersion. A user does not need to prepare the aqueous dispersion due to increased storage stability for the user, and thus handleability is increased.

Setting the total molar ratio to 80/20 or less causes a state in which the self-micelle-like aggregates and the hydrophilic-rich aggregates coexist. In such a case, the thickening property and the thixotropic property are increased to some extent as compared with a conventional hydrophilic fumed silica, which is stable, but hardly provides the increased thickening property and thixotropic property. That is, new use applications with the emphasis on both the stability and the low viscosity can be developed. For example, new use applications can be developed for cream, an emulsified cosmetic, or the like, where the low thickening property and thixotropic property, and the excellent stability are both required. Further, a user does not need to prepare the aqueous dispersion due to the excellent storage stability at the user side, and thus handleability is increased.

Setting the total molar ratio to 20/80 or more and 80/20 or less causes a state in which the self-micelle-like aggregates predominantly exist. This state is stable and exhibits certain levels of the viscosity and the thixotropic property. Further, this state is homogenous without including the hydrophobic-rich aggregates and the hydrophilic-rich aggregates.

Thus, it becomes possible to provide a stable and homogenous function-imparting agent mainly focusing on the thickening property and the thixotropic property, allowing development of a very large number of use applications. Variations in functions in the same batch and variations between batches can also be reduced. As use applications that can take advantage of such characteristics, a very large number of use applications, such as a cosmetic, a functional coating material, a functional coating agent, an adhesive, and a sealant for construction, may be mentioned. Further, the desirable levels of the thickening property and the thixotropic property can be imparted in each use application by appropriately adjusting the total molar ratio. Further, a user does not need to prepare the aqueous dispersion due to the excellent storage stability at the user side, and thus handleability is increased.

The Pickering emulsion according to the present invention is the oil-in-water type emulsion, which contains the composite particle groups including the self-micelle-like aggregates formed by the fumed silica particle groups as the secondary aggregate and the oil inside the self-micelle-like aggregates, and in each composite particle group, the surface of the oil drop is coated with the self-micelle-like aggregates. The self-micelle-like aggregates are stable in the aqueous dispersion and this state is essentially preserved in the Pickering emulsion.

The stable, homogenous, and highly reproducible Pickering emulsion can be produced by coating the oil droplet with the self-micelle-like aggregates.

The secondary aggregate of the fumed silica exists reportedly in a size of about 10 $\mu$m in a normal condition. However, in the oil-in-water type emulsion according to the present invention, the aggregated matters of the spherical silica particles are used for coating and each aggregated matter normally has a size of about 0.1 $\mu$m to several $\mu$m. Thus, a small portion of the secondary aggregates includes the primary aggregates and may possibly include the intermediate aggregates at the stage of shifting from the primary aggregates to the secondary aggregates. Alternatively, it is also conceivable that the secondary aggregates are more densely formed than the secondary particles existing in a usual state.

The oil contained in the oil-in-water type emulsion composition of the present invention is not limited, and any kind of oil component may be used. For example, silicone, mineral oil, synthetic oil, vegetable oil, animal oil, and the like can be used.

Silicone is a preferred oil since it provides excellent properties in a wide range of industrial and cosmetic use applications. The type is not limited. Examples thereof may include linear polysiloxanes (such as dimethylpolysiloxane, methylphenylpolysiloxane, and diphenylpolysiloxane), cyclic polysiloxanes (such as octamethylcyclotetrasiloxane, decamethylcyclopentasiloxane, and dodecamethylcyclohexasiloxane), silicone resins which form a three-dimensional network structure, silicone rubber, various modified polysiloxanes (such as amino-modified polysiloxane, polyether-modified polysiloxane, alkyl-modified polysiloxane, and fluorine-modified polysiloxane), alkenyl-modified polysiloxanes, hydrogen polysiloxane, and acrylic silicones.

The viscosity of the oil is also not limited. Also, as long as it is fluid, the oil may contain solid components. In addition, the oil may be a flowable rubber or rubber-containing material, or a curable rubber composition which is cured after formation of an emulsion to form a rubber.

In each composite particle present in the oil-in-water type emulsion according to the present invention, a self-micelle-like aggregate of fumed silica particles coats an oil droplet. The secondary aggregate coats an oil droplet with one layer or forms multiple layers. Also, a part of an oil droplet may not be coated with fumed silica particles.

The secondary aggregate being in contact with an oil droplet is partly embedded into an oil droplet. The embedding degree varies depending on the polarity degree of oil and the molar ratio between hydrophilic groups and hydrophobic groups on the surface of the fumed silica secondary aggregate. When the polarity of an oil droplet is close to that of the secondary aggregate, for example, when silicone is an oil droplet and the polarity on the surface of secondary particles is close to that of silicone, the affinity thereof is high, and thus the embedding degree becomes high. Conversely, when a difference in polarity is large, the embedding degree is low.

It is said that an optimum contact angle determined by inorganic particles, an oil droplet, and water is important for achieving a stable Pickering emulsion. In the oil-in-water type emulsion of the present invention, the embedding degree of the secondary aggregate of fumed silica particles into an oil droplet is mainly determined by the above-described mechanism. However, primary aggregates having a high hydrophobic degree in the secondary aggregate can move and be oriented toward an oil droplet by an appropriate shear force in the process of completing an emulsion, and thus the polarity can be tilted in secondary particles. There is the possibility that an optimum contact angle is obtained by spontaneously adjusting the above-described tilted degree to determine the embedding degree for achieving a most stable emulsion.

Also, the secondary aggregate can be moved by appropriate shear force even after an emulsion has been formed. The secondary aggregates can move on the surface of an oil droplet to homogenize an interval and a layered state. Furthermore, some of the secondary aggregates can move to other composite particles.

The secondary aggregates of fumed silica particles may coat an oil droplet entirely or partly. Possible examples of an index representing the coating degree include the thickness of a silica layer and the result of morphological observation. However, the coating amount is adopted here as a more practical index of the coating degree for controlling properties. The properties refer to properties (such as stability) as an emulsion. Also, when isolated composite particles are used, the properties refer to properties such as non-blocking properties of the isolated composite particles.

The amount of the coating layer of silica particles on the surface can be parameterized as a weight of fumed silica particles per unit area on the surface of an oil droplet.

In the present invention, the amount of the silica coating layer can be controlled by the processing amount of silica particles relative to a surface area, which is calculated from a particle size of composite particles. This amount is preferably $2.0 \times 10^{-9}$ to $3.2 \times 10^{-6}$ kg/m$^2$. When less than $2.0 \times 10^{-9}$ kg/m$^2$, the composite particles are blocked in water or after isolation. When more than $3.2 \times 10^{-6}$ kg/m$^2$, friction of the composite particles increases. This causes unfavorable phenomena in specific uses. For example, the sense of coarseness in cosmetics is caused. Also, the contamination due to excessive silica occurs in an emulsion or after the isolation of particles.

In the oil-in-water type emulsion according to the present invention, the particle size of each composite particle is influenced by the mass ratio of fumed silica particles relative to the total amount of oil. The higher the ratio, the smaller the particle size. When the amount of the silica coating layer falls within the above-described preferable range, the particle size is almost about 10 μm, regardless of the type of oil. Although this particle size is larger than that of particles in an emulsion emulsified with a common organic surfactant, stability and homogeneity can be achieved with this particle size. When applied to various use applications, a particle size of about 100 μm or less is suitable for a Pickering emulsion. When the particle size exceeds 100 μm, the stability of an emulsion decreases. Furthermore, when isolated composite particles are used, the size of powder can be sensed by touch in use applications such as cosmetics. Therefore, it is not preferable from the viewpoint of the sense during use.

In the oil-in-water type emulsion according to the present invention, a variation of the particle size among composite particle groups is small. This is because the surface of an oil droplet is coated with the self-micelle-like aggregates of fumed silica particles. Since the self-micelle-like aggregates in an aqueous dispersion are homogeneous, the action on the oil droplet is homogenized. Accordingly, a variation of the particle size decreases.

The oil-in-water type emulsion according to the present invention can be produced using a stirrer similar to an apparatus used for producing an aqueous dispersion of fumed silica particles.

For coating the surface of an oil droplet with the self-micelle-like aggregates of fumed silica particles, a method of forming self-micelle-like aggregates of fumed silica particles and then pouring an oil needs to be adopted. This is because if an oil is first charged, the self-micelle-like aggregates of fumed silica particles are not formed, and thus an oil-in-water type emulsion as an intended Pickering emulsion is not obtained.

Therefore, the producing method includes, as the first stage, preparing fumed silica particle groups at a secondary aggregation level such that a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) becomes 20/80 or more and 80/20 or less. For achieving the total molar ratio within a prescribed value range, the molar ratio is adjusted with fumed silica as a raw material, in the same manner as the method for generating the self-micelle-like aggregates in the aqueous dispersion.

Next, the second stage includes a stage of adding the prepared fumed silica particle groups at a secondary aggregation level to water and applying a shear force at a prescribed shear speed to promote the exchange at a primary aggregation level among the fumed silica particle groups at a secondary aggregation level and/or the orientation at a primary aggregation level in the fumed silica particles at a secondary aggregation level, whereby a difference between an average value of the total mole number ratio and a molar ratio represented by (the number of moles of surface hydrophilic groups/the number of moles of surface hydrophobic groups) in each of the secondary particles constituting the fumed silica particle groups becomes a prescribed value or less. Thus, an aqueous dispersion containing self-micelle-like aggregates of fumed silica particle groups at a secondary aggregation level is generated. For causing rearrangement, it is suitable to apply shear force at 7500 s$^{-1}$ or more.

Next, the third stage includes adding an oil to the generated aqueous dispersion to form an emulsion. At this time, a shear force is applied at an appropriate speed in a stirrer. For homogeneously coating the surface of an oil droplet with the self-micelle-like aggregates to homogeneously form a structure as a composite particle, a time for applying a shear force is appropriately required. The shear speed does not need to be 7500 s$^{-1}$ or more which causes rearrangement. Usually, about several thousands s$^{-1}$ is sufficient.

For preparing a stable and homogeneous Pickering emulsion, it is important to mainly generate self-micelle-like aggregates by setting a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) to fall within a prescribed value range. The state (for example, the embedding degree of the secondary aggregates into an oil droplet at the interface) of the self-micelle-like aggregates in each composite particle containing an oil droplet coated with the self-micelle-like aggregates preferably does not have a variation to the extent possible. When the variation is small, the connection among the self-micelle-like aggregates is strengthened. Therefore, the composite particles are likely to become further stable. This stabilizes the composite particle groups as a whole, and reduces a variation of the state among the composite particles.

The variation among the secondary aggregates is reduced at the second stage of the above-described producing method. When the variation of the hydrophobization degree (hydrophilization degree) among the secondary aggregates is small, the variation of the embedding degree among the secondary aggregates being in contact with an oil droplet in each self-micelle-like aggregate is also small. When the variation of the hydrophobization degree (hydrophilization degree) among the secondary aggregates is roughly within ±60% of an average value of the variation of the hydrophobization degree (hydrophilization degree) for all secondary aggregates, a stable oil-in-water type emulsion is formed.

The variation among the secondary aggregates can also be reduced by using, as a raw material fumed silica particle, a particle including a hydrophilic group hydrophobized at a specific hydrophobization rate, without applying a shear force for causing rearrangement. However, the production cost of the method is high. Such a specialized silica particle may not be used, because a simple producing method is enabled only by using as a raw material a commercially available inexpensive hydrophilic fumed silica particle and hydrophobic fumed silica particle to adjust the mass ratio to a prescribed total molar ratio.

The variation of the state among the composite particles can also be reduced, without significantly reducing the variation of the hydrophobization degree (hydrophilization degree) among the secondary aggregates, by promoting the orientation at a primary aggregation level in the fumed silica particles at a secondary aggregation level. When orientation proceeds, the concentration of hydrophobic groups near a portion being in contact with the interface of an oil droplet in the secondary aggregate increases, while the concentration of hydrophilic groups near a portion being in contact with an aqueous phase increases. Accordingly, both the embedding into an oil droplet and the affinity to an aqueous phase are efficiently achieved.

When the variation of the hydrophobization degree (hydrophilization degree) among the secondary aggregates decrease, and the orientation at a primary aggregation level in fumed silica particles at a secondary aggregation level proceeds, a synergistic effect can be exhibited, and more stable and homogeneous composite particles can be prepared.

In terms of the variation among the composite particles, the setting of the mass ratio between fumed silica particles and an oil is also important. As described above, the coating thickness with silica particles on the surface of an oil droplet desirably does not vary among the composite particles. For this reason, it is desirable to set the mass of silica particles to within the above-described stable coating amount range relative to the mass of oil.

When silica is also used as a functional particle in the oil-in-water type emulsion or the isolated composite particles according to the present invention, the mass ratio of an oil to silica particles is also important.

A method for calculating a variation between an average value of the molar ratio of (hydrophilic groups/hydrophobic groups) for the secondary aggregates and a molar ratio of each secondary aggregate in an aqueous dispersion of fumed silica particles and an oil-in-water type emulsion was found in the present invention, and therefore will be described below. In the present invention, an oil droplet is coated with the self-micelle-like aggregates of fumed silica particles to obtain an oil-in-water type emulsion (Pickering emulsion), and secondary particles have been rearranged in the process of preparing the aqueous dispersion. Therefore, it is considered that the above-described molar ratio variation is the same between at the stage of an emulsion and at the previous stage of completing an aqueous dispersion. A method for calculating the above-described molar ratio variation based on this premise will be described.

A total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) is appropriately assigned between 0 and 100%. The viscosity of an aqueous dispersion which has been applied with a shear force at a speed of 7500 $s^{-1}$ or more to sufficiently cause rearrangement for achieving a variation of 0 is measured. Also, the viscosity of an aqueous dispersion which has not been rearranged and has achieved a maximum variation is measured. Then, the value of the viscosity at a low shear speed point is divided by the value of the viscosity at a high shear speed point. The calculated value is a conventionally known thixotropy index (hereinafter, abbreviated as a TI value). Since an aqueous dispersion having a large variation contains a hydrophobic-rich aggregate at a high ratio, a TI value, that is, thixotropy, increases.

A percent value of a variation between an average value of the molar ratio of (hydrophilic groups/hydrophobic groups) for the secondary aggregates and a molar ratio of each secondary aggregate is obtained by removing composite particles after an emulsion has been prepared, analyzing the surface of the secondary aggregates of silica particles by SEM-EDX (energy dispersive X-ray spectroscopy, an analysis method using a SEM {scanning electron microscope} with functions such as elemental analysis), and calculating an average value of the ratio and a variation %. In the observation of the surface of the composite particles in the oil-in-water type emulsion according to the present invention by SEM, it is confirmed that a spherical aggregate, which is considered to be a secondary aggregate, is embedded into an oil droplet. Therefore, the spherical aggregate is analyzed as a secondary aggregate. An SiOH group and an SiOSiMe$_3$ group are presumed to be present on the surface of silica particles. Accordingly, the amounts of a carbon atom and an oxygen atom are analyzed by element mapping.

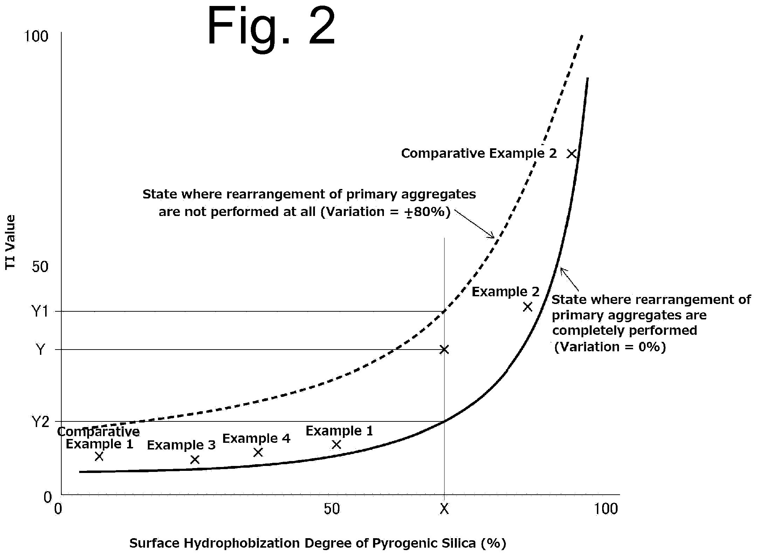

Fig. 2 illustrates a plot of a surface hydrophobization degree (the total molar ratio in a raw material) of an original aqueous dispersion vs. a TI value when the variation by SEM-EDX is 0%, and a plot of the aqueous dispersion when the variation is 80%. Detailed data will be described in Examples.

As illustrated in Fig. 2, a variation Z% when a TI value at a hydrophobization degree X is Y is calculated by proportional distribution according to formula (1), utilizing Y1 that is a TI value at a hydrophobization degree X on a TI calibration curve for a variation $\alpha$%, and Y2 that is a TI value at a hydrophobization degree X on a TI calibration curve for a variation $\beta$% ($\leq \alpha$%).

$$Z = \beta + (\alpha - \beta) \times (Y - Y2)/(Y1 - Y2) \qquad (1)$$

When two calibration curves are previously created with master aqueous dispersions, a variation % value at an optional hydrophobization degree X can be calculated by merely measuring a TI value. That is, it was found that stability and homogeneity can be checked by an extraordinarily low-cost and simple method.

When a predetermined TI value is previously set for achieving an acceptable variation degree depending on the use application and purpose, and a measured TI value is the TI value or less, the stability and homogeneity of an aqueous dispersion and an oil-in-water type emulsion obtained with the aqueous dispersion can be selected.

It is noted that the evaluation of a TI of an emulsion is difficult. Since the effects of the oil droplet type, particle size, and the like are expressed, a practical parameter cannot be put in order.

The stability and homogeneity of an aqueous dispersion can also be analyzed by measuring a zeta potential, because a peak corresponding to an aggregation state of fumed silica particles appears.

As described above, the oil-in-water type emulsion according to the present invention which achieves stability and homogeneity with required levels depending on its use applications and purpose can be designed by, for example, selecting a raw material, setting a hydrophobization degree, comparing the amounts of silica and oil, and preliminary checking stability and homogeneity.

As already described, for obtaining a stable and homogeneous oil-in-water type emulsion (Pickering emulsion), an aqueous dispersion as an intermediate of an emulsion is necessary to be formed as a self-micelle-like dispersion of fumed silica particles at a stage of preparing an aqueous dispersion. Furthermore, a TI value needs to be checked for completing a stable and homogeneous aqueous dispersion having a small variation. Such a state is basically maintained in an emulsion.

In addition, it is important to performing auxiliary shearing in a process of preparing an emulsion for further promoting the orientation of primary aggregates in secondary aggregates.

Furthermore, an appropriate time is necessary after appropriate shearing has been performed, in order to homogeneously coat an oil droplet with silica particles at a certain thickness.

According to the oil-in-water type emulsion (Pickering emulsion) obtained in the present invention, which includes an oil droplet coated with fumed silica particles, stability and homogeneity are reliably achieved at a stage of generating an aqueous dispersion. Since auxiliary stabilization is further performed, stability and homogeneity significantly increase. Furthermore, favorable reproducibility, high reliability, and no restriction on the selection and form of the oil enable various use applications to be developed.

According to the present invention, the homogeneity of the composite particles in the aqueous dispersion can be checked by a simple method at a stage of an intermediate of an oil-in-water type emulsion. Therefore, an emulsion as a final product can reliably achieve stability and homogeneity. Furthermore, yields for eliminating defective products significantly improve. Also, the design for that purpose is possible.

When an emulsion is an intended product, various known use applications are expected to be improved, and novel use applications are expected to be developed. Such use applications include a use application of formulating it to various cosmetics for causing various silicone as an oil to adhere with the hair and skin so that various effects are provided, a use application of requiring an extraordinarily precise and reproducible coat as a coating film for various electronic materials and a coating film for airbags, and a coat requiring significant heat resistance and homogeneity such as a release agent for die casting.

According to the present invention, an oil-in-water type emulsion reliably achieving stability and homogeneity is generated, and the coating degree of the silica layer can be controlled. Therefore, the isolated composite particles can have reliability.

When water is removed to isolate the composite particles, and silica serves as a functional particle, the function of reducing hygroscopicity due to the action of an oil or rubber elasticity is provided in a known use application of a reinforcing agent for plastics. When the oil is a cured silicone rubber which is soft inside and hard outside, a use application such as a powder for cosmetics which imparts the sense of looseness and is easy to spread is conceivable. Since the variation among the composite particles is small in these use applications, the function improves in the use applications.

When silica is not a functional particle, silicone rubber particles, which have a favorable particle size and shape uniformity, improve performance when formulated in various paints and plastics. Also, a new product form as a dry emulsion is conceivable in which the composite particles are appropriately returned into water for emulsification.

EXAMPLES

Next, the present invention will be described by way of examples. Note that the present invention is not limited by the examples.

Further, the contents of the fumed silica used in Examples are as follows and the evaluation methods of the aqueous dispersions and the oil-in-water type emulsions including the fumed silica particles of the present invention and the compositions in Comparative Examples are performed as follows.

<Details of fumed silica>

Silicas used in Examples and Comparative Examples of the present invention are silicas 1 to 3 shown in Table 1, which are fumed silica (dry silica). The details are as illustrated in Table 1.

[Table 1]

| <Details of silica> | | | |
|---|---|---|---|
| | Hydrophilic pyrogenic silica ("silica 1") | Hydrophobic pyrogenic silica ("silica 2") | Hydrophilized hydrophobic pyrogenic silica ("silica 3") |
| BET surface area | 200±30 | 120±40 | 200±30 |
| Molar ratio of surface hydrophilic groups vs. surface hydrophobic groups in entire particle at raw material stage | 90/10 | 10/90 | 70/30 |

<Measuring method of viscosity and TI (thixotropy index)>

For a silica aqueous dispersion obtained in each of Examples and Comparative Examples, the viscosity under the condition of 25°C and 7.5 s$^{-1}$ and the viscosity under the condition of 25°C and 350 s$^{-1}$ were measured using a B-type viscometer (manufactured by Brookfield, model: DV-II+Pro). The viscosity measured under the condition of 25°C and 7.5 s$^{-1}$ was divided by the viscosity measured under the condition of 25°C and 370 s$^{-1}$ to derive a TI (thixotropy index) value.

<Measuring method of particle size >

The particle size of the oil-in-water type emulsion particles obtained in Examples and Comparative Examples was measured by using a laser diffraction scattering particle size distribution analyzer (product name "LS-230") manufactured by Beckman-Coulter, Inc. and represented as an average particle size.

<Evaluation method of stability and homogeneity>

An evaluation method of the stability of the aqueous dispersion and the oil-in-water type emulsions obtained in each of Examples and Comparative Examples was performed as follows: thirty grams of each sample was placed in a 50 ml screw vial and the presence of precipitation was examined after the sample was stored for one month at the room temperature. For the emulsions, the presence of creaming was also examined. Further, the homogeneity was visually examined for the presence of a large granular material and turbidity at a peripheral part.

Evaluation standard;

AA: homogenous with no creaming or precipitation at all, A: less homogeneous with limited creaming and precipitation, B: slightly inhomogeneous with moderate creaming and precipitation, C: inhomogeneous with creaming and precipitation

<Analysis of variation of hydrophobization degree of composite particle by SEM-EDX measurement>

The molar ratio between surface hydrophobic groups and surface hydrophilic groups in the secondary aggregates of silica particles present on the surface of the composite particles in the oil-in-water type emulsion obtained in each of Examples and Comparative Examples was analyzed by element mapping through surface analysis by SEM-EDX.
The quantitative technique by element mapping for an existence ratio of a carbon atom and an oxygen atom on the particle surface was performed by the following method.
First, an emulsion was dried at 150°C for 3 hours or more to obtain dried emulsion particles in which a water content has completely evaporated, i.e. composite particles.

Quantitative determination by SEM-EDX was performed by the following method.

A noble metal antistatic layer having a thickness of about 10 nm was formed on the dried emulsion particles using a sputter apparatus or a deposition apparatus. Thus, a sample for SEM was prepared.

This sample for SEM was set on a JSM-5600LV SEM-EDX apparatus manufactured by JEOL, and observed at a magnification of about 5,000 to 10,000 times. The EDX point analysis near the center of the sample was performed by integration at a sample inclination angle of 0° and an acceleration voltage of 20 kV to obtain a ratio between a carbon atom and an oxygen atom on the surface of the composite particles.

On the basis of this carbon atom ratio value, a variation % of the surface hydrophobization degree of each secondary aggregate unit with respect to the average value was calculated.

<Discussion on relationship between variation value of hydrophobization degree among secondary aggregates by SEM-EDX measurement and TI>

With respect to the hydrophobization degree of raw material in each of Examples and Comparative Examples, master aqueous dispersions in which the variation of the hydrophobization degree at a secondary aggregate level of a raw material is 0 and ±80% were prepared by adjusting a shear speed. The presence of an intended product was checked by SEM-EDX, and the TI values of the aqueous dispersions at that time were measured. The measured TI values were plotted in relation to the hydrophobization degree. Thus, calibration curves were created.

On this relationship diagram, the TI value of the aqueous dispersion obtained in each of Examples and Comparative Examples was plotted. The consistency between a variation value obtained by proportional distribution based on the position of the value between the above-described two calibration curves and an actual value obtained by a value actually measured by SEM-EDX was checked.

The consideration result is illustrated in Fig. 2.

<Method for producing aqueous dispersion and oil-in-water type emulsion in Examples and Comparative Examples>

In each of Examples and Comparative Examples, an aqueous dispersion was obtained on the basis of preparation amounts and production conditions shown in Table 2. Further, an oil-in-water type emulsion was produced on the basis of the preparation amounts shown in the same table with the use of the resulting aqueous dispersion.

Evaluation results of the oil-in-water type emulsions were shown in Table 3.

<Example 1>

As a first stage, 2.5 g of hydrophilic fumed silica ("silica 1") and 2.5 g of hydrophobic fumed silica ("silica 2") were charged in a 500mL stainless steel beaker. To this, 45 g of deionized water was charged and the mixture was stirred at 3,000 rpm for 2 minutes by using Ultraturrax to obtain a silica aqueous dispersion. The shearing speed in this stirring was about 11,000 s$^{-1}$.

Next, as a second stage, 50 g of dimethylpolysiloxane having a viscosity of 1,000 mPa·s (available under the name of "AK1000" from Wacker Chemie AG (Munich, Germany)) was charged, and the mixture was stirred at 1,500 rpm for 2 minutes to obtain an oil-in-water type emulsion having a low viscosity in white color. The shearing speed in this stirring was about 5,700 s$^{-1}$.

<Example 2>

An aqueous dispersion and an oil-in-water type emulsion having a low viscosity in white color were obtained in the same manner as in Example 1 except that 1 g of the silica 1 and 4 g of the silica 2 were charged.

<Example 3>

An aqueous dispersion and an oil-in-water type emulsion having a low viscosity in white color were obtained in the same manner as in Example 1 except that 4 g of the silica 1 and 1 g of the silica 2 were charged.

<Example 4>

Five grams of partially hydrophobized hydrophilic fumed silica ("silica 3") was charged in a 500mL stainless steel beaker. To this, 45 g of deionized water was charged and the mixture was stirred at 3,000 rpm for 2 minutes by using Ultraturrax to obtain a silica aqueous dispersion. Next, 50 g of methylpolysiloxane having a viscosity of 1,000 mPa·s (available under the name of "AK1000 from Wacker Chemie AG (Munich, Germany)) was charged, and the mixture was stirred at 1,500 rpm for 2 minutes to obtain an oil-in-water type emulsion having a low viscosity in white color.

<Example 5>

An aqueous dispersion and an oil-in-water type emulsion having a low viscosity in white color were obtained in the same manner as in Example 1 except that the stirring time at the second stage was one minute.

<Example 6>

An aqueous dispersion and an oil-in-water type emulsion having a low viscosity in white color were obtained in the same manner as in Example 1 except that the stirring at the second stage was performed at 500 rpm. The shearing speed in this stirring was about 1,900 s$^{-1}$.

<Example 7>

An aqueous dispersion and an oil-in-water type emulsion having a low viscosity in white color were obtained in the same manner as in Example 1 except that the stirring time at the first stage was one minute.

<Comparative Example 1>

Five grams of hydrophilic fumed silica ("Silica 1") was charged into a 500 mL stainless steel beaker. To this, 45 g of deionized water was added, and the mixture was stirred at 3,000 rpm for 2 minutes using Ultraturrax to obtain a silica aqueous dispersion.
Subsequently, 50 g of methylpolysiloxane having a viscosity of 1,000 mPa·s (available under the name of "AK1000" from Wacker Chemie AG (Munich, Germany)) was charged, and the mixture was stirred at 1,500 rpm for 2 minutes. However, oil-in-water properties were not sufficient.

<Comparative Example 2>

An entirely white liquid was obtained in the same manner as in Comparative Example 1 except that 5 g of hydrophobic fumed silica ("silica 2") was added instead of 5 g of hydrophilic fumed silica ("silica 1"). Oil-in-water properties in a state of being separated into an aqueous phase which was a continuous phase and an oil phase which was a dispersed phase was not sufficient.

<Comparative Example 3>

In a 500 mL stainless steel beaker, 2.5 g of hydrophilic fumed silica ("Silica 1") and 2.5 g of hydrophobic fumed silica ("Silica 2") were charged. Into this liquid, added was 45 g of deionized water, and the mixture was stirred at 500 rpm for 2 minutes using Ultraturrax to obtain a silica aqueous dispersion. The shearing speed in this stirring was about 1,900 s$^{-1}$.
Subsequently, 50 g of methylpolysiloxane having a viscosity of 1,000 mPa·s (available under the name of "AK1000" from Wacker Chemie AG (Munich, Germany)) was charged, and the mixture was stirred at 1,500 rpm for 2 minutes to obtain an entirely white liquid. However, oil-in-water properties in a state of being separated into an aqueous phase which was a continuous phase and an oil phase which was a dispersed phase was not sufficient.

<Comparative Example 4>

As a first stage, 50 g of dimethylpolysiloxane having a viscosity of 1,000 mPa·s (available under the name of "AK1000" from Wacker Chemie AG) was charged in a 500mL stainless steel beaker. To this, 45 g of deionized water was charged, and the mixture was stirred at 1,900 rpm for 2 minutes by using Ultraturrax to obtain an oil-in-water type emulsion. The shearing speed in this stirring was about 5,700 s$^{-1}$.
Subsequently, as a second stage, 2.5 g of hydrophilic fumed silica ("silica 1") and 2.5 g of hydrophobic fumed silica ("silica 2") were charged and stirred at 3,000 rpm for 2 minutes. The shearing speed in this stirring was about 5,700 s$^{-1}$.
An entirely white liquid was obtained, but oil-in-water properties in a state of being separated into an aqueous phase which was a continuous phase and an oil phase which was a dispersed phase was not sufficient.

<Comparative Example 5>

into a 500mL stainless steel beaker, 2.5 g of hydrophilic fumed silica ("silica 1"), 2.5 g of hydrophobic fumed silica ("silica 2") and 50 g of dimethylpolysiloxane having a viscosity of 1,000 mPa·s ("AK1000" from Wacker Chemie AG) were simultaneously charged. To this, 45 g of deionized water was added, and the mixture was stirred at 3,000 rpm for 2 minutes using Ultraturrax. The shearing speed in this stirring was about 5,700 s$^{-1}$.

An entirely white liquid was obtained, but oil-in-water properties in a state of being separated into an aqueous phase which was a continuous phase and an oil phase which was a dispersed phase was not sufficient.

[Table 2]

| | | | Example 1 | Example 2 | Example 3 | Example 4 | Example 5 | Example 6 | Example 7 |
|---|---|---|---|---|---|---|---|---|---|
| <Preparation amount and production condition of pyrogenic silica aqueous dispersion and oil-in-water type emulsion> | | | | | | | | | |
| Mass parts | Hydrophilic pyrogenic silica ("silica 1") | | 2.5 | 1 | 4 | | 2.5 | 2.5 | 2.5 |
| | Hydrophobic pyrogenic silica ("silica 2") | | 2.5 | 4 | 1 | | 2.5 | 2.5 | 2.5 |
| | Partially hydrophobized hydrophilic pyrogenic silica ("silica 3") | | | | | 5 | | | |
| | Water | | 45 | 45 | 45 | 45 | 45 | 45 | 45 |
| | Dimethyl polysiloxane (Wacker AK 1000) | | 50 | 50 | 50 | 50 | 50 | 50 | 50 |
| Molar ratio of (surface hydrophilic groups/surface hydrophobic groups) in entire silica particle at raw material stage | | | 50/50 | 26/74 | 74/26 | 70/30 | 50/50 | 50/50 | 50/50 |
| Charging order | First stage | | Silica | Silica | Silica | Silica | Silica | Silica | Silica |
| | Second Stage | | Oil | Oil | Oil | Oil | Oil | Oil | Oil |
| Shear speed ($s^{-1}$)/stirring time (min) (first stage) | | | 11,000/2 | 11,000/2 | 11,000/2 | 11,000/2 | 11,000/2 | 11,000/2 | 11,000/1 |
| Shear speed ($s^{-1}$)/stirring time (min) (second stage) | | | 5,70012 | 5,700/2 | 5,700/2 | 5,700/2 | 5,700/1 | 1,900/2 | 5,700/2 |

(continued)

| | | Comparative Example 1 | Comparative Example 2 | Comparative Example 3 | Comparative Example 4 | Comparative Example 5 |
|---|---|---|---|---|---|---|
| Mass parts | Hydrophilic pyrogenic silica ("silica 1") | 5 | | 2.5 | 2.5 | 2.5 |
| | Hydrophobic pyrogenic silica ("silica 2") | | 5 | 2.5 | 2.5 | 2.5 |
| | Partially hydrophobized hydrophilic pyrogenic silica ("silica 3") | | | | | |
| | Water | 45 | 45 | 45 | 45 | 45 |
| | Dimethyl polysiloxane (Wacker AK 1000) | 50 | 50 | 50 | 50 | 50 |
| Molar ratio of (surface hydrophilic groups/surface hydrophobic groups) in entire silica particle at raw material stage | | 90/10 | 10/90 | 50/50 | 50/50 | 50/50 |
| Charging order | First stage | Silica | Silica | Silica | Oil | Simultaneous |
| | Second Stage | Oil | Oil | Oil | Silica | |
| Shear speed ($s^{-1}$/stirring time (min)) (first stage) | | 11,000/2 | 11,000/2 | 1,900/2 | 5,700/2 | 11,000/2 |
| Shear speed ($s^{-1}$/stirring time (min)) (second stage) | | 5,700/2 | 5,700/2 | 5,700/2 | 11,000/2 | - |

[Table 3]

| <Evaluation result of pyrogenic silica aqueous dispersion and oil-in-water type emulsion> | | | | | | | | | |
|---|---|---|---|---|---|---|---|---|---|
| | | Example 1 | Example 2 | Example 3 | Example 4 | Example 5 | Example 6 | Example 7 |
| Aqueous dispersion | Stability | A | A | A | A | A | A | A |
| | TI value | 5 | 61 | 4 | 4.5 | 5 | 5 | 5 |
| Oil-in-water type emulsion | Oil-in-water properties | A | A | A | A | A | A | A |
| | Stability, homogeneity | AA | AA | AA | AA | A | A | AA |
| | Particle size (μm) | 15-20 | 15~20 | 15~20 | 15~20 | 15~20 | 15~20 | 15~20 |
| | Variaton (maximum %) of hydrophobization degree of silica secondary aggregate by SEM-EDX from average value | ±5 | ±6 | ±6 | ±6 | ±5 | ±5 | ±5 |

| | | Comparative Example 1 | Comparative Example 2 | Comparative Example 3 | Comparative Example 4 | Comparative Example 5 |
|---|---|---|---|---|---|---|
| Aqueous dispersion | Stability | A | C | C | - | |
| | TI value | 2 | 80 | 26 | | |
| Oil-in-water type emulsion | Oil-in-water properties | C | C | C | C | C |
| | Stability, homogeneity | C | C | C | C | C |
| | Particle size (μm) | Unmeasurable | Unmeasurable | Unmeasurable | Unmeasurable | Unmeasurable |
| | Variation (maximum %) of hydrophobization degree of silica secondary aggregate by SEM-EDX from average value | ±8 | ±8 | ±80 | Unmeasurable | Unmeasurable |

<Summary of production results of aqueous dispersions and oil-in-water type emulsions>

As shown in Tables 2 and 3, at a stage of the fumed silica as a raw material, when the ratio between the total number of moles of (the surface hydrophilic groups of the entire particle groups obtained from each of the silica particle groups/the total number of moles of the surface hydrophobic groups of the entire particle groups obtained from each of the particle groups) was in a range of 26/74 to 74/26, the stable aqueous dispersions and oil-in-water type emulsions were produced. However, when the molar ratio was 90/10, while the aqueous dispersion was stable, the stable oil-in-water type emulsion was not produced. When the molar ratio was 10/90, neither the aqueous dispersion nor the emulsion was stable.

Even if the molar ratio was 50/50, neither the aqueous dispersion nor the emulsion was stable with adopting the low shearing speed.

When the shearing speed was sufficiently large, the variations in the degree of surface hydrophobicity at the secondary aggregate level became sufficiently small in both cases; however, when the shearing speed was not sufficient, the variations became large.

It is believed that the oil was taken in with the shearing speed that was significantly lower than that applied for producing the aqueous dispersion, and thus the self-micelle-like aggregates in the aqueous dispersion were shifted to the oil-in-water type emulsion (the Pickering emulsion) in almost the same state.

Since the observations by SEM-EDX are believed to substantially reflect the status of the self-micelle-like aggregates of the aqueous dispersion, it is believed that stable and homogeneous aqueous dispersions and emulsions were produced in the respective examples.

It was confirmed that homogenization between self-micelle-like aggregates is an important factor for suppression of variation between composite particles.

It was confirmed that the stable emulsion could not be produced by simultaneously mixing the silica, water, and the oil, or by mixing the silica after mixing water and the oil. Further, it was confirmed that it was enough to perform the primary shearing for the aqueous dispersion, and the auxiliary shearing for the emulsion. The formation of the self-micelle-like aggregates in the aqueous dispersion can be speculated on the basis of these observations.

It was confirmed that, for reliably achieving the stability of the emulsion, the total molar ratio and variations thereof vary in accordance with the aggregating property of the silica.

It was confirmed that, regarding the shearing, considering that the rearrangement is required for the stability of the emulsion, although there is no restriction on the shearing time as long as the shearing speed is at a predetermined level or higher, the shearing speed and the shearing time are involved in adjusting the specifications of the composite particles.

<Discussion on SEM-EDX result and TI result>

Fig. 2 illustrates two calibration curves as a solid line and a dashed line. Master aqueous dispersions contain silica 1 and silica 2 used in Examples and Comparative Examples. The solid calibration curve was obtained by plotting a measurement result of a TI value for an aqueous dispersion in which the variation of the hydrophobization degree of the secondary aggregate by SEM-EDX was 0, that is, an aqueous dispersion having been completely rearranged. The dashed curve was obtained by plotting a measurement result of a TI value for an aqueous dispersion in which the variation was ±80%, that is, an aqueous dispersion not having been rearranged at all. In this diagram, TI values in Examples 1 to 4 and Comparative Examples 1 and 2 are marked with mark ×. In the SEM-EDX measurement, each master aqueous dispersion was transformed into an oil-in-water type emulsion by the same method as Example 1, and composite particles were analyzed.

Table 4 shows production conditions, variation values, and TI measurement values of the master aqueous dispersions.

From the position of mark × in Fig. 2, a variation Z% when the TI value at a hydrophobization degree X is Y was calculated by proportional distribution according to formula (1), utilizing Y1 that is a TI value at a hydrophobization degree X on a TI calibration curve for a variation $\alpha$%, and Y2 that is a TI value at a hydrophobization degree X on a TI calibration curve for a variation $\beta$% ($\leq \alpha$%).

$$Z = \beta + (\alpha - \beta) \times (Y - Y2)/(Y1 - Y2) \quad (1)$$

The calculation results are illustrated in Table 4.

Table 5 compares the variation values calculated by SEM-EDX and the variation values calculated from the calibration curves of a TI according to formula (1) in Examples 1 to 4 and Comparative Examples 1 and 2. Favorable consistency was exhibited.

This demonstrated that a variation value can be calculated from these calibration curves by measuring a TI value at an optional molar ratio of the hydrophobization degree at a raw material stage.

Also, it was found that a stable and homogeneous aqueous dispersion can be prepared and designed by the simple method of a TI, without sophisticated and complicated direct observation through an electron microscope.

[Table 4]

| <Production condition and TI value of master aqueous dispersion (variation 0%)> | | | | | | | | |
|---|---|---|---|---|---|---|---|---|
| | | 1 | 2 | 3 | 4 | 5 | 6 |
| Mass parts | Hydrophilic pyrogenic silica ("silica 1") | 2.5 | 1 | 4 | | 5 | |
| | Hydrophobic pyrogenic silica ("silica 2") | 2.5 | 4 | 1 | | | 5 |
| | Water | 45 | 45 | 45 | 45 | 45 | 45 |
| Molar ratio of (surface hydrophilic groups/surface hydrophobic groups) in entire silica particle at raw material stage | | 50/50 | 26/74 | 74/26 | 70/30 | 90/10 | 10/90 |
| Shear speed $(s^{-1})$/stirring time (min) (first stage) | | 11,000/5 | 11,000/5 | 11,000/5 | 11,000/5 | 11,000/5 | 11,000/5 |
| Variation % by SEM-EDX | | 0 | 0 | 0 | 0 | 0 | 0 |
| TI value | | 9 | 34 | 5 | 7 | 5 | 63 |
| <Production condition and TI value of master aqueous dispersion (variation $\pm 80\%$)> | | | | | | | | |
| | | 7 | 8 | 9 | 10 | 11 | 12 |
| Mass parts | Hydrophilic pyrogenic silica ("silica 1") | 2.5 | 1 | 4 | | 5 | |
| | Hydrophobic pyrogenic silica ("silica 2") | 2.5 | 4 | 1 | | | 5 |
| | Water | 45 | 45 | 45 | 45 | 45 | 45 |
| Molar ratio of (surface hydrophilic groups/surface hydrophobic groups) in entire silica particle at raw material stage | | 50/50 | 26/74 | 74/26 | 70/30 | 90/10 | 10/90 |
| Shear speed $(s^{-1})$/stirring time (min) (first stage) | | 1,900/2 | 1,900/2 | 1,900/2 | 1,900/2 | 1,900/2 | 1,900/2 |
| Variation % by SEM-EDX | | 80 | 80 | 80 | 80 | 80 | 80 |
| T\| value | | 26 | 69 | 17 | 21 | 14 | 93 |

[Table 5]

| <Consistency between variation value of hydrophobicity among composite particles analyzed by SEM-EDX and variation value calculated from TI calibration curves> | | | | | | |
|---|---|---|---|---|---|---|
| | Example 1 | Example 2 | Example 3 | Example 4 | Comparative Example 1 | Comparative 2 |
| Variation value (%) of hydrophobic degree among composite particles analyzed by SEM-EDX | $\pm 5$ | $\pm 6$ | $\pm 6$ | $\pm 6$ | $\pm 8$ | $\pm 8$ |

(continued)

| <Consistency between variation value of hydrophobicity among composite particles analyzed by SEM-EDX and variation value calculated from TI calibration curves> | | | | | | |
|---|---|---|---|---|---|---|
| | Example 1 | Example 2 | Example 3 | Example 4 | Comparative Example 1 | Comparative 2 |
| Variation value calculated from TI calibration curves (%) | ±5 | ±6 | ±6 | ±6 | ±8 | ±8 |

<Discussion on generation of self-micelle-like aggregate>

(1) A fact that a stable oil-in-water type emulsion including silica, water, and an oil was formed by adding the oil after an aqueous dispersion of silica and water has been formed, but a stable oil-in-water type emulsion was not formed by simultaneously adding silica, water and an oil or by previously adding the oil and water and thereafter adding silica.

(2) A fact that when an oil was added after an aqueous dispersion including silica and water had been formed, a shear force was necessary to be applied at a prescribed shear speed or more during the formation of an aqueous dispersion for forming a stable and homogeneous aqueous dispersion, and a shear force was not always necessary to be applied for forming an oil-in-water type emulsion by adding an oil because of the stable and homogeneous aqueous dispersion.

(3) A fact that, regardless of the types of silica and an oil, an oil-in-water type emulsion can be formed by adding the oil after an aqueous dispersion including silica and water has been formed.

(4) A fact that a stable emulsion can be formed when two parameters are within a prescribed range, one parameter is a total mole number ratio represented by (the total number of moles of surface hydrophilic groups of the entire silica particle groups obtained from each of the silica particle groups/the total number of moles of surface hydrophobic groups of the entire silica particle groups obtained from each of the silica particle groups) and the other parameter is a difference between an average value of the total mole number ratio and a molar ratio between the number of moles of surface hydrophilic groups and the number of moles of surface hydrophobic groups in each of the secondary particles constituting the silica particle group. In this case, the solubility of silica into water, attributable to mainly contained hydrophilic-rich aggregates as the silica particle groups, and the expression of thixotropy, attributable to mainly contained hydrophobic-rich aggregates as the silica particle groups, had decreased.

(5) A fact that when a composite particle in a stable oil-in-water type emulsion is observed through a SEM, the surface of the composite particle contains bulk substances having a relatively spherical shape and homogeneous size which coat the surface of an oil droplet.

(6) A fact that the variation of the particle size or the like among formed composite particle groups is small, and the shear speed in an aqueous dispersion form influences the specification of the composite particle.

From the above-described facts in Examples, the application with a shear force at a prescribed shear speed during the formation of an aqueous dispersion with silica and water enables the formation of aggregates which are mainly neither hydrophilic-rich aggregates nor hydrophobic-rich aggregates. Also, a stable oil-in-water type emulsion is formed by adjusting the total mole number ratio and the variation of the total mole number ratio and thereafter adding an oil, regardless of the types of silica and the oil as a raw material. Therefore, self-micelle-like aggregates are reliably estimated to be formed during the formation of an aqueous dispersion, in that the application of shear force induces the rearrangement among silica aggregates and/or the orientation within a silica aggregate such that the hydrophilicity density is high outside, and the hydrophobicity density is high inside.

INDUSTRIAL APPLICABILITY

The aqueous dispersion including the inorganic particles of the present invention is stable and homogenous and expresses functions of imparting the thickening property, the thixotropic property, and the like, and thus reliability in the conventional use applications such as modifying a coating material, a cosmetic, and various resins is significantly improved and new use applications such as a functional coating material, a functional coating agent, an adhesive, and a sealant for construction can be developed. Further, the aqueous dispersion can be used as an intermediate for producing the stable and homogenous Pickering emulsion.

Further, in the oil-in-water type emulsion (the Pickering emulsion) of the present invention, the stability and homogeneity are very high, there is no limitations on the types of the oil, the homogeneity can be examined at the intermediate stage, the production is performed by the simple and low-cost method, and design for obtaining the stable and homogenous oil-in-water type emulsion can be performed. Thus, the reliability in the conventional use applications can be significantly improved, and use applications in a cosmetic with high functionality and use applications in a new field requiring high requested properties and reliability such as an electronic material and an air bag can be expected.

When the composite particles of the oil-in-water type emulsion of the present invention are isolated, the stability and homogeneity are reliably maintained, thus making it possible to provide the composite particles, which are easy to handle, have small variations in properties, and have high reliability. Thus, new use applications in a cosmetic, additives for various resins, and the like can be expected. Further, the isolated composite particles can be returned to the emulsion again by adding water. Thus, significant improvements in costs in production storage and transportation can be expected as compared with those using the composite particles in the emulsion state, thereby causing tremendous impact on industries.

BRIEF DESCRIPTION OF DRAWING

Fig. 1 is a schematic view enlarged for clarification, illustrating what aggregates can be formed when fumed silica particles are dispersed in water according to an embodiment.
Fig. 2 illustrates thixotropy index curves with a variation of the total mole number ratio as a parameter, a surface hydrophobic degree of pyrogenic silica as a horizontal axis, and a thixotropy index value as a vertical axis.

Reference Signs List

10     hydrophilic-rich primary aggregate
12     hydrophobic-rich primary aggregate
14     secondary aggregate
16     space

**Claims**

1. An aqueous dispersion of inorganic particles, including inorganic particle groups dispersed in an aggregation state in water, wherein a total mole number ratio represented by (a total number of moles of surface hydrophilic groups of the entire inorganic particle groups obtained from each of the inorganic particle groups/a total number of moles of surface hydrophobic groups of the entire inorganic particle groups obtained from each of the inorganic particle groups) is within a value range of a prescribed lower limit value or more depending on aggregating properties of inorganic particles, whereby the inorganic particle groups contain self-micelle-like aggregates and hydrophobic-rich aggregates.

2. An aqueous dispersion of inorganic particles, including inorganic particle groups dispersed in an aggregation state in water, wherein a total mole number ratio represented by (a total number of moles of surface hydrophilic groups of the entire inorganic particle groups obtained from each of the inorganic particle groups/a total number of moles of surface hydrophobic groups of the entire inorganic particle groups obtained from each of the inorganic particle groups) is within a value range of a prescribed upper limit value or less depending on aggregating properties of inorganic particles, whereby the inorganic particle groups contain self-micelle-like aggregates and hydrophilic-rich aggregates.

3. An aqueous dispersion of inorganic particles, including inorganic particle groups dispersed in an aggregation state in water, wherein a total mole number ratio represented by (a total number of moles of surface hydrophilic groups of the entire inorganic particle groups obtained from each of the inorganic particle groups/a total number of moles of surface hydrophobic groups of the entire inorganic particle groups obtained from each of the inorganic particle groups) is within a value range of a prescribed lower limit value or more and a prescribed upper limit value or less depending on the aggregating properties of inorganic particles, whereby the inorganic particle groups contain self-micelle-like aggregates.

4. The aqueous dispersion of inorganic particles according to any one of claims 1 to 3, wherein the inorganic particle groups contain silica.

5. The aqueous dispersion of inorganic particles according to any one of claims 1 to 3, wherein the inorganic particle groups contain fumed silica, the prescribed lower limit value is 20/80, and the prescribed upper limit value is 80/20.

6. A method for producing an aqueous dispersion of fumed silica particles, including fumed silica particle groups dispersed in an aggregation state in water, wherein a total mole number ratio represented by (a total number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) is set so as to become 20/80 or more at a raw material stage of the fumed silica particle groups, and the aqueous dispersion of the fumed silica particles contains self-micelle-like aggregates and hydrophobic-rich aggregates.

7. A method for producing an aqueous dispersion of fumed silica particles, including fumed silica particle groups dispersed in an aggregation state in water, wherein a total mole number ratio represented by (a total number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) is set so as to become 80/20 or more at a raw material stage of the fumed silica particle groups, and the aqueous dispersion of the fumed silica particle contains self-micelle-like aggregates and hydrophilic-rich aggregates.

8. A method for producing an aqueous dispersion of fumed silica particles, including fumed silica particle groups dispersed in an aggregation state in water, wherein a total mole number ratio represented by (a total number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) is set so as to become 20/80 or more and 80/20 or less at a raw material stage of the fumed silica particle groups, and the aqueous dispersion of the fumed silica particle contains self-micelle-like aggregates.

9. A method for producing an aqueous dispersion containing a self-micelle-like aggregate group by fumed silica particle groups at a secondary aggregation level, comprising
a stage of preparing fumed silica particle groups at a secondary aggregation level such that a total mole number ratio represented by (a total number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) is 20/80 or more and 80/20 or less, and
a stage of adding the prepared fumed silica particle groups at the secondary aggregation level to water to form self-micelle-like aggregates of the fumed silica particle groups at the secondary aggregation level.

10. The method for producing an aqueous dispersion of fumed silica particles according to any one of claims 8 and 9, comprising a process of applying a shear force that is sufficient for causing exchange of the fumed silica particle groups at the primary aggregation level among the fumed silica particle groups at the secondary aggregation level while the fumed silica particle groups are dispersed in water.

11. An oil-in-water type emulsion, comprising composite particle groups each having an oil contained in self-micelle-like aggregates of fumed silica particle groups at a secondary aggregation level in which a total mole number ratio represented by (a total number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) is 20/80 or more and 80/20 or less, wherein each of the composite particle groups contain an oil droplet coated with the self-micelle-like aggregates.

12. The oil-in-water type emulsion according to claim 11, wherein a difference between an average value of the total mole number ratio and a molar ratio represented by (a number of moles of surface hydrophilic groups/a number of moles of surface hydrophobic groups) in each of the fumed silica particle groups is a prescribed value or less, whereby homogenization among the self-micelle-like aggregates is achieved.

13. The oil-in-water type emulsion according to any one of claims 11 and 12, wherein the oil is a curable rubber composition or a rubber-containing oil which maintains fluidity.

14. A method for producing an oil-in-water type emulsion, including:

a stage of preparing fumed silica particle groups at a secondary aggregation level such that a total mole number ratio represented by (a total number of moles of the entire fumed silica particle groups/the total number of moles of surface hydrophobic groups of the entire fumed silica particle groups obtained from each of the fumed silica particle groups) a total mole number ratio represented by (the total number of moles of surface hydrophilic groups of the entire fumed silica particle groups obtained from each of the fumed silica particle groups/the total number of moles of surface hydrophobic groups of the entire fumed silica particle groups obtained from each of the fumed silica particle groups) becomes 20/80 or more and 80/20 or less;

a stage of adding the prepared fumed silica particle groups at the secondary aggregation level to water and applying a shear force at a prescribed shear speed to promote exchange at a primary aggregation level among the fumed silica particle groups at the secondary aggregation level and/or orientation at the primary aggregation level in the fumed silica particles at the secondary aggregation level, thereby generating an aqueous dispersion containing self-micelle-like aggregates of the fumed silica particle groups at the secondary aggregation level such that a difference between an average value of the total mole number ratio and a molar ratio represented by (the number of moles of surface hydrophilic groups/the number of moles of surface hydrophobic groups) in each of the secondary particles constituting the fumed silica particle groups becomes a prescribed value or less; and

a stage of adding an oil to the generated aqueous dispersion to form an emulsion, whereby the oil-in-water type emulsion contains composite particle groups including the oil inside the self-micelle-like aggregates formed of the fumed silica particle groups at the secondary aggregation level.

15. The method for producing an oil-in-water type emulsion according to [14], wherein the stage of generating an aqueous dispersion containing self-micelle-like aggregates of fumed silica particle groups includes a stage of creating master thixotropy index curves by plotting thixotropy indices of two master aqueous dispersions which have the molar ratio variation close to 0 and a maximum value with respect to a total mole number ratio while previously knowing a molar ratio variation that is a difference between an average value of the total mole number ratio and a molar ratio represented by (a number of moles of surface hydrophilic groups/a number of moles of surface hydrophobic groups) in each of the same type of fumed silica particle groups, measuring a thixotropy index of an aqueous dispersion prepared at an optional total mole number ratio, comparing the measured thixotropy index to the master thixotropy index curves at the total molar ratio to calculate the molar ratio variation of the prepared aqueous dispersion by proportional distribution, and setting the molar ratio variation to a prescribed value or less.

16. The method for producing an oil-in-water type emulsion according to any one of claims 14 and 15, wherein the stage of forming an emulsion further includes applying an auxiliary shear force at a prescribed shear speed to promote the orientation at the primary aggregation level in the fumed silica particle groups at the secondary aggregation level.

17. The method for producing an oil-in-water type emulsion according to any one of claims 14 to 16, further comprising adjusting a mass ratio between the oil and the fumed silica particle groups to adjust a coating degree and/or a coating thickness of the self-micelle-like aggregates on a surface of an oil droplet in the composite particle groups.

18. A method for designing an oil-in-water type emulsion aiming at achieving stability of an emulsion, comprising:

a stage of obtaining a master thixotropy index curve of a master aqueous dispersion including fumed silica particle groups dispersed in an aggregation state in water, in which a total mole number ratio represented by (a total number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) and a molar ratio variation that is a difference between an average value of the total mole number ratio represented by (a number of moles of surface hydrophilic groups of the entire fumed silica particle groups obtained from each of the fumed silica particle groups/a total number of moles of surface hydrophobic groups of the entire fumed silica particle groups obtained from each of the fumed silica particle groups) and a molar ratio represented by (a number of moles of surface hydrophilic groups/a number of moles of surface hydrophobic groups) in each of the fumed silica particle groups are previously known;

a stage of measuring a thixotropy index of an aqueous dispersion including fumed silica particle groups of the same type as and the same total mole number ratio as the master aqueous dispersion dispersed in water in an aggregation state;

a stage of comparing the measured thixotropy index with the master thixotropy index curve to evaluate the molar ratio variation of the aqueous dispersion; and

a stage of adding an oil to the aqueous dispersion having been evaluated for the molar ratio variation, to generate an oil-in-water type emulsion containing composite particle groups containing the oil inside self-micelle-like aggregates of the fumed silica particle groups at a secondary aggregation level.

19. The method for designing an oil-in-water type emulsion according to claim 18, wherein the stage of evaluating the molar ratio variation of an aqueous dispersion includes creating master thixotropy index curves by plotting thixotropy indices of two master aqueous dispersions which have the molar ratio variation close to 0 and a maximum value with respect to a total mole number ratio while previously knowing a molar ratio variation that is a difference between an average value of the total mole number ratio and a molar ratio represented by (the number of moles of surface hydrophilic groups/the number of moles of surface hydrophobic groups) in each of the same type of fumed silica particle groups, measuring a thixotropy index of an aqueous dispersion prepared at an optional total mole number ratio, comparing the measured thixotropy index with the master thixotropy index curves at the total molar ratio, and calculating the molar ratio variation of the prepared aqueous dispersion by proportional distribution.

20. The method for designing an oil-in-water type emulsion according to any one of claims 18 and 19, comprising previously grasping the molar ratio variation of the master aqueous dispersion by carbon analysis and oxygen analysis of the composite particle groups through an electron microscope.

# Fig. 1

Hydrophilic-Rich Aggregate
(Soluble, Low Viscosity,
Small Thixotropic Property)

Self-Micelle-Like
Aggregate

Hydrophobic-Rich
Aggregate
(High Viscosity, Large
Thixotropic Property)

Homogeneity

Stability

EP 3 656 738 A1

# Fig. 2

State where rearrangement of primary aggregates are not performed at all (Variation = ±80%)

Comparative Example 2 ✕

State where rearrangement of primary aggregates are completely performed (Variation = 0%)

Example 2 ✕

Example 1 ✕

Example 4 ✕

Example 3 ✕

Comparative Example 1 ✕

TI Value — 100, 50, Y1, Y, Y2, 0

Surface Hydrophobization Degree of Pyrogenic Silica (%) — 0, 50, 100

## INTERNATIONAL SEARCH REPORT

| International application No. |
|---|
| PCT/JP2017/028922 |

A. CLASSIFICATION OF SUBJECT MATTER
*C01B33/18*(2006.01)i

According to International Patent Classification (IPC) or to both national classification and IPC

B. FIELDS SEARCHED

Minimum documentation searched (classification system followed by classification symbols)
C01B33/00-33/193, C08L83/04

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)
JSTPlus(JDreamIII), JST7580(JDreamIII), JSTChina(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2005-126722 A  (Wacker Chemie GmbH),<br>19 May 2005 (19.05.2005),<br>claims; paragraphs [0001], [0013]; examples<br>& US 2005/0107520 A1<br>claims; paragraphs [0002], [0019]; examples<br>& EP 1526153 A1          & DE 10349082 A1<br>& KR 10-2005-0039607 A   & CN 1624024 A | 1-14,16,17<br>13<br>15,18-20 |
| X<br>Y<br>A | JP 2016-121230 A  (Wacker Asahikasei Silicone<br>Co., Ltd.),<br>07 July 2016 (07.07.2016),<br>claims; paragraphs [0015] to [0017], [0026],<br>[0032]<br>& WO 2016/102384 A1<br>claims; paragraphs [0015] to [0017], [0026],<br>[0032]<br>& CN 107109061 A          & KR 10-2017-0092635 A | 1-12,14,16,<br>17<br>13<br>15,18-20 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 October 2017 (27.10.17) | 14 November 2017 (14.11.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.

PCT/JP2017/028922

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2012-500766 A  (Wacker Chemie AG.),<br>12 January 2012 (12.01.2012),<br>claims; paragraphs [0025], [0030], [0042],<br>[0060] to [0062]; examples<br>& US 2011/0139035 A1<br>claims; paragraphs [0026], [0031], [0043],<br>[0063] to [0065]; examples<br>& WO 2010/020582 A2      & DE 102008041466 A1<br>& CN 102131733 A          & KR 10-2011-0058831 A | 1-12,14,16,<br>17<br>13<br>15,18-20 |
| Y | JP 10-175816 A  (Dow Corning Toray Silicone Co.,<br>Ltd.),<br>30 June 1998 (30.06.1998),<br>claims<br>& US 5928660 A<br>claims | 13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004203735 A
- JP 2000095632 A
- JP 2008031487 A

**Non-patent literature cited in the description**

- *J. Jpn. Soc. Colour Mater.,* 2016, vol. 89 (6), 203
- *J. Chem. Phys.,* 2006, vol. 124, 241104
- Master's thesis by Tomoyuki Suzuki, Department of Chemistry for Materials, Graduate School of Engineering (Master's Course). Mie University, 2010